(19) Europäisches Patentamt
European Patent Office
Office européen des brevets

(11) **EP 2 481 391 B1**

(12) **EUROPÄISCHE PATENTSCHRIFT**

(45) Veröffentlichungstag und Bekanntmachung des Hinweises auf die Patenterteilung:
**05.07.2017   Patentblatt 2017/27**

(51) Int Cl.:
***A61K 6/083*** (2006.01)

(21) Anmeldenummer: **11194478.1**

(22) Anmeldetag: **20.12.2011**

(54) **Dentale provisorische Suprakonstruktionen sowie Materialien zu ihrer Herstellung und entsprechende Verfahren**

Dental provisional superstructures and materials for producing same and corresponding method

Superstructures dentaires provisoires ainsi que matériaux utilisés pour leur fabrication et procédé correspondant

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.01.2011   DE 102011003289**

(43) Veröffentlichungstag der Anmeldung:
**01.08.2012   Patentblatt 2012/31**

(73) Patentinhaber: **VOCO GmbH**
**27472 Cuxhaven (DE)**

(72) Erfinder:
• **Maletz, Reinhard**
  **27472 Cuxhaven (DE)**

• **Blömker, Tobias**
  **27472 Cuxhaven (DE)**
• **Hoffmann, Klaus-Peter**
  **27474 Cuxhaven (DE)**
• **Plaumann, Manfred Thomas**
  **27476 Cuxhaven (DE)**

(74) Vertreter: **Eisenführ Speiser**
**Patentanwälte Rechtsanwälte PartGmbB**
**Postfach 10 60 78**
**28060 Bremen (DE)**

(56) Entgegenhaltungen:
**WO-A2-2008/037753      DE-A1- 3 708 618**
**DE-A1- 19 941 829      US-A1- 2003 219 606**

EP 2 481 391 B1

**Beschreibung**

Die vorliegende Erfindung betrifft ein dentales Gemisch und ein dentales Mehrkomponentensystem zur Herstellung des Materials einer provisorischen Suprakonstruktion für ein dentales Implantat oder eines Kerns für eine provisorische Suprakonstruktion für ein dentales Implantat durch Polymerisationshärtung, zur spezifischen Anwendung in einer therapeutischen Behandlung.

Die Erfindung betrifft zudem die entsprechenden provisorischen Suprakonstruktionen und Kerne selbst, welche durch Polymerisationshärtung aus den erfindungsgemäßen dentalen Gemischen oder den erfindungsgemäßen dentalen Mehrkomponentensystemen herstellbar sind. Die vorliegende Erfindung betrifft auch einen entsprechenden provisorischen Zahnersatz, der neben einer erfindungsgemäßen provisorischen Suprakonstruktion auch ein dentales Implantat und ein vorzugsweise metallisches Abutment umfasst. Gegenstand der vorliegenden Erfindung ist auch ein Kit zur Herstellung einer Mehrzahl von erfindungsgemäßen provisorischen Suprakonstruktionen für ein dentales Implantat oder einer Mehrzahl von erfindungsgemäßen Kernen für provisorische Suprakonstruktionen für ein dentales Implantat. Ein entsprechendes Verfahren zur Herstellung einer provisorischen Suprakonstruktion oder eines Kerns ist ebenfalls Gegenstand der vorliegenden Erfindung. Die Erfindung betrifft auch ein Verfahren zur provisorischen Versorgung eines dentalen Implantats für therapeutische und/oder kosmetische Zwecke, wobei erfindungsgemäße provisorische Suprakonstruktionen eingesetzt werden. Beschrieben wird auch die Verwendung eines dentalen Gemisches oder eines dentalen Mehrkomponentensystems zum Herstellen einer provisorischen Suprakonstruktion oder eines Kerns für eine solche provisorische Suprakonstruktion.

Der Gegenstand der Erfindung ist in den beigefügten Ansprüchen definiert.

Sämtliche Aspekte der vorliegenden Erfindung stehen dabei im Zusammenhang mit dem Einsatz von dentalen Implantaten und insbesondere den Verfahren des progressive bone loading.

In der modernen Zahnmedizin stellt der Einsatz von Zahnimplantaten eine aufwändige, ansonsten jedoch zuverlässige und auch ästhetisch ansprechende Behandlungsmethode zur Versorgung von Zahnlücken dar.

Gegenüber den alternativen Möglichkeiten des Zahnersatzes wie festen nichtimplantatgestützten Brücken oder herausnehmbaren Prothesen weisen Implantate deutliche Vorteile auf; insbesondere belasten sie den Kieferknochen in fast der gleichen Weise wie der ursprüngliche Zahn. Ein zahnärztliches Implantat (dentales Implantat) ist eine künstliche Zahnwurzel meist in der technischen Ausgestaltung einer Schraube, die in den Kieferknochen eingepflanzt wird, wenn der Zahn mitsamt seiner Wurzel verlorengegangen ist. Durch das Verwachsen des Implantats mit dem Knochen übernimmt das Implantat fast die gleichen Funktionen wie die ursprüngliche Zahnwurzel und leitet ebenfalls Kräfte in den Knochen ein, wodurch der Knochen auf Zug beansprucht wird. Durch diese Belastung des Kieferknochens wird der Knochenstoffwechsel aktiv stimuliert und unterstützt, so dass der Kieferknochen erhalten bleibt. Der zahnlose, nur vom Zahnfleisch bedeckte Kieferknochen unter Totalprothesen hingegen wird durch die fehlende, auf Zug wirkende Krafteinleitung in den Knochen und zusätzlich oberflächlich wirkenden Druck falsch belastet und baut sich über Jahre teilweise langsam, teilweise rasant immer weiter ab. Durch den fortschreitenden Knochenabbau verändert sich das gesamte Knochenlager, was nach Jahren zu großen Problemen führt.

Zahnmedizinische Implantate werden daher in den Kieferknochen eingesetzt, um nach dem Einheilen eine im Vergleich zu einer Versorgung ohne Implantate bessere prothetische Versorgung für den Patienten zu ermöglichen und den ortständigen Knochen zu erhalten. So werden bei zahnlosen Patienten dadurch vielfach Totalprothesen vermieden, da der Patient entweder mit auf den Implantaten festsitzendem oder kombiniert festsitzend-herausnehmbarem Zahnersatz versorgt werden kann. Bei Lückengebissen kann das Beschleifen von gesunden Zähnen zur Herstellung von konventionellen (nichtimplantatgestützten) Brücken vermieden werden.

Der implantologische Eingriff ist recht umfangreich und umfasst im Wesentlichen die folgenden Schritte:

- Einsetzen des Implantats in den Kieferknochen

- Einheilung des Implantats in den Knochen

- Freilegung des Implantates

- Abdrucknahme mit speziellen Abdruckhilfen

- Herstellung des Zahnersatzes (der Suprakonstruktion)

- Einprobe des von der Zahntechnik angefertigten Zahnersatzes

- Einsetzen des endgültigen Zahnersatzes (endgültige Versorgung des Implantats).

Unter "Suprakonstruktion" wird im Rahmen des vorliegenden Textes ein Element verstanden, das direkt oder indirekt mit einem dentalen Implantat zu verbinden oder verbunden ist und dazu vorgesehen ist, vollständig oder fast vollständig in die Mundhöhle zu ragen. In der Regel ist die Suprakonstruktion ein Zahnersatz. Eine Suprakonstruktion kann beispielsweise eine Krone, eine Brücke (bzw. ein Teil einer Brücke) oder eine herausnehmbare Prothese (bzw. ein Teil einer herausnehmbaren Prothese) sein. Der Begriff "endgültig" wie etwa im Kontext "endgültige Suprakonstruktion" oder "endgültiger Zahnersatz" bezeichnet im Rahmen des vorliegenden Textes ein Element, das nicht von vornherein dazu bestimmt ist, nach einer gewissen Zeitspanne durch ein anderes Element ersetzt zu werden. Im Gegensatz dazu wird unter "Provisorium" im Rahmen des vorliegenden Textes ein Element verstanden, das von vornherein dazu bestimmt ist, zu einem späteren Zeitpunkt durch ein anderes Element ersetzt zu werden. Das Adjektiv "provisorisch" ist entsprechend zu verstehen. In der Regel ist ein Verbindungselement zwischen Implantat und Suprakonstruktion vorgesehen, ein sogenanntes Abutment (Aufbau). Ein gegebenenfalls vorhandenes Abutment wird im Rahmen des vorliegenden Textes nicht zur Suprakonstruktion gezählt. Unter "Verbinden" einer provisorischen Suprakonstruktion mit einem dentalen Implantat ist sowohl ein Positionieren der Suprakonstruktion direkt auf dem Implantat (ohne Verwendung eines Abutments) als auch ein Positionieren der Suprakonstruktion auf einem Abutment, das auf dem Implantat positioniert ist (indirektes Verbinden) zu verstehen.

Im oben angegebenen Behandlungsablauf beträgt die Einheilphase in der Regel zwischen 2 und 6 Monaten. Hierbei kommt es zur sogenannten Osseointegration des Implantates. Das bedeutet, dass Knochen auf die Implantatoberfläche aufwächst und das Implantat fest im Knochen verankert wird. Es kommt dabei aber noch nicht zu einer optimalen architektonischen Ausrichtung der Knochenstruktur, welche Kräfte aufnehmen und weiterleiten kann. Im oben angegebenen Behandlungsablauf wird bei der anschließenden Freilegung des Implantates und durch die darauf anschließend erfolgende Versorgung der Implantate mit der prothetischen Arbeit (der Suprakonstruktion) eine schlagartige einhundertprozentige Übertragung von Kräften, also eine ohne Übergangsphase einsetzende vollständige Übertragung der beim Kauen erzeugten Kräfte, auf das vorher durch Kräfte unbelastete Implantat und die unbelastete umgebende Knochenstruktur bewirkt. Der Nachteil des oben angegebenen Behandlungsablaufes ist also die spontan einsetzende, einhundertprozentige Belastung der Implantate und umgebenden Knochenstruktur, welche eventuell nicht der Knochensituation um das Implantat herum entspricht und den Knochen in manchen Situationen überbelastet.

Um eine gute Osseointegration zu erreichen, benötigen der Knochen und die darin vorhandenen Zellen Zeit, auf der Oberfläche des Implantates anzuwachsen und umgebenden Knochen umzubauen. Bisher ging man davon aus, dass die Zeit und die Qualität des Knochens für die Einheilungsgeschwindigkeit und auch für den Erfolg der Osseointegration verantwortlich sind. Es wird diskutiert, ob auf das Implantat einwirkende physiologische Kräfte die Osseointegration fördern. Physiologische Kräfte würden die erstehende Knochenstruktur nur soweit belasten, wie es der bis dahin eingetretene Heilungsprozess erlaubt. Durch eine solche physiologische Krafteinleitung und eine daraus resultierende Verbesserung des Einheilvorganges lässt sich vermutlich die Einheilzeit verkürzen.

Wie lange ohne Anwendung einer solchen Technik der physiologischen Krafteinleitung die Einheilzeit sein soll, richtet sich nach anerkannten Klassifizierungen der Dichte und Struktur des Knochens, die Empfehlungen für bestimmte Einheilzeiten vorgeben. Im Allgemeinen sind dies für den Oberkiefer 6 Monate und für den Unterkiefer 3 Monate, da hier verschiedene Knochenstrukturen und Qualitäten zu finden sind.

Eine für die erfolgreiche Implantation weitere wichtige Voraussetzung ist, dass das Implantat nach dem Einbringen in den Knochen eine sogenannte Primärstabilität besitzt. Das heißt, dass es direkt nach dem Einbringen nicht locker sitzen darf, sondern absolut, d.h. im maximal möglichen Ausmaß, fest im Knochen verankert ist, was man auch mit Hilfe von Geräten, wie den unter dem Namen Periotest® vertriebenen, messen kann. Interessanterweise wird das Implantat nach initialer Primärstabilität oft nach etwa 14 Tagen etwas lockerer, um dann wieder fester zu werden. Dies wird durch Knochenab- und Umbauvorgänge erklärt. Wahrscheinlich ist das Implantat innerhalb dieser Zeit gegenüber äußeren Krafteinflüssen sehr sensibel.

In den letzten Jahren sind neue Überlegungen zu den nach dem Einsetzen eines Implantats erforderlichen Einheilzeiten gemacht worden. Vor allem dem Wunsch der Patienten folgend, nicht mehr so lange bis zur endgültigen Versorgung warten zu müssen, wurde angefangen, mit verkürzten Einheilzeiten oder ganz ohne Einheilzeiten und entsprechend der soeben genannten Alternative mit sofortiger (direkt nach der Implantation oder innerhalb von 24 bis 36 Stunden danach) provisorischer oder endgültiger Versorgung des Implantates und Belastung zu arbeiten.

Studien zur Sofortbelastung von Implantaten im Vergleich zu der herkömmlichen Methode sind widersprüchlich. Es gibt Studien, die zeigten, dass die erreichte Einheilung schlechter bei der Sofortimplantation war (M. Lorenzoni, C. Pertl, K. Zhang, W. Wegschneider, Clin. Oral Implants Res., 2003, 14 (3) 273-279); andere Studien fanden keinen Unterschied (P. Quinlan et al., Int. J. Oral Maxillofac Implants 2005, 20 (3), 360-370) oder kamen zu der Einschränkung, dass die Patienten genau auszuwählen und die Belastungskräfte auf die Implantate möglichst gering zu halten sind (G. Romanos, J. Oral Implantol. 2004, 30 (3), 189-197).

Die Misserfolge bei der Sofortbelastung scheinen durch zu frühe und zu starke Kraftübertragungen auf die Implantat-Knochenstruktur, die in diesen Fällen gerade im Aufbau und Umbau ist, herzurühren. Wahrscheinlich kann dieses System bestimmte physiologische Kräfte tolerieren, eventuell wirken diese sogar stimulierend; werden sie aber zu groß, reißt die Toleranz ab und es kommt zu keiner Osseointegration, sondern zu einer bindegewebigen Einscheidung des Implantates.

Die herkömmliche Methode mit den vergleichsweise langen Einheilzeiten hat den Nachteil, dass es nicht langsam und kontinuierlich zu diesen Umbauvorgängen kommt, da der Kieferknochen zunächst über einen längeren Zeitraum nicht belastet wird (keine Okklusion mit dem Gegenkiefer) und später eine plötzliche relative Vollbelastung durch das Provisorium bzw. die endgültige Versorgung (Suprakonstruktion) erfährt, welche auch zu Implantatverlusten führen kann.

Die Methode der Sofortbelastung ist nachteilig, denn sie birgt nach bisherigen Erkenntnissen das Risiko, dass es zu Überbelastungen der sich umbauenden Knochen-Implantatgrenze kommt, welche in dieser frühen Phase der Einheilung dann den Verlust des Implantates bedeuten können.

Diesen Erkenntnissen folgend wurde die sogenannte (nachfolgend auch als "klassisch" bezeichnete) Methode der progressiven Kraftbelastung (progressive bone loading) beschrieben (R. Appleton et al., Clin. Oral Implants Res. 2005, 16 (2), 161-167). Dabei müssen die Kräfte langsam auf den Knochen übertragen werden. Diese steigende Kraftübertragung nennt man "progressives Belasten". Hierbei wird zunächst ein Provisorium (eine provisorische Suprakonstruktion) auf Kunststoffbasis eingesetzt, das zunächst sicherstellt, dass der provisorische Zahnersatz (das Provisorium) nicht mit seinem Antagonisten okkludiert. Der Patient ist in dieser Zeit angehalten, nur flüssige oder breiartige Nahrung zu sich zu nehmen. Das Provisorium wird schließlich so abgeändert, dass es in mehreren Schritten immer dichter an den antagonistischen Zahn gebracht wird, bis es schließlich in Okklusion mit dem Gegenkiefer steht. Der Kieferknochen soll sich während dieses Zeitraums der steigenden Belastung anpassen, bis schließlich die endgültige Versorgung erfolgt.

Das dadurch erfolgende Knochentraining soll langsame, durch physiologische und reduzierte Kräfte induzierte Umbauvorgänge der Knochenarchitektur bewirken. Gerade diese Knochenarchitektur ist wichtig, um Kräfte im Knochen physiologisch zu verteilen, da dies ein Grundprinzip der Natur und des Knochens ist.

Der provisorische Zahnersatz wird im Stand der Technik aus herkömmlichen dentalen Prothesenkunststoffen beträchtlicher Härte angefertigt. Hierzu werden bevorzugt radikalisch polymerisierbare Acrylate eingesetzt. Diese Systeme sind in der Literatur erschöpfend beschrieben, so z.B. in der EP 270915, in der EP 630640, in der US 6063830, in der US 5548001, in der US 4617327, sowie in der EP 0677286.

Neben den auf der Acrylatchemie basierenden Systemen wurden Spiroorthoester und polycyclische Ketallactone zur Herstellung von Dentalmaterialen vorgeschlagen (US 4387215). Die DE 19506222 beschreibt kationisch polymerisierbare Materialien auf der Basis von Oxetan- und Oxacyclobutan-Derivaten. Weiterhin sind bicycloaliphatische 2-Methylen-1,3-dioxepane aus der DE 4439485 bekannt. Die US 5665839 offenbart radikalisch polymerisierbare Oxathiepane, die DE 19612004 beschreibt radikalisch polymerisierbare multifunktionelle Vinylcyclopropanderivate, während die DE 102004002178 Monomeren vorschlägt, die durch ringöffnende Metathese vernetzen können.

Nachteil der vorstehend beschriebenen, "klassischen" progressive bone loading Methode ist die Tatsache, dass Patienten nur Brei essen dürfen und dass es aufgrund der Tatsache, dass dann keine funktionellen Kaukräfte entstehen, zu keiner kontrollierbaren reduzierten Krafteinleitung im Vergleich zu einer Situation bei funktionaler Vollbelastung kommt. Falls der Patient jedoch Speisen zu sich nehmen sollte, die ein Kauen erfordern, sind die auf das Implantat einwirkenden Kaukräfte nicht zu kontrollieren.

Es gibt im Stand der Technik bereits Bemühungen, provisorische Suprakonstruktionen für dentale Implantate anzugeben, mit deren Hilfe die mit der Verwendung herkömmlicher provisorischer Suprakonstruktionen jeweils verbundenen Nachteile minimiert werden können, insbesondere auch die Nachteile, die mit der "klassischen" progressive bone loading Methode verbunden sind. So offenbart beispielsweise die WO 2008/037753 A2 elastische provisorische Suprakonstruktionen für dentale Implantate, die aus Materialien mit einem Elastizitätsmodul von weniger als 300 MPa bestehen. Die in der WO 2008/037753 A2 offenbarten provisorischen Suprakonstruktionen basieren auf speziellen härtbaren Ausgangsmaterialien, insbesondere Siliconen umfassend mehratomige vernetzbare Gruppen oder gegebenenfalls substituierte vernetzbare Polyether. Die in der WO 2008/037753 A2 vorgeschlagenen härtbaren Ausgangsmaterialien haben sich in der Praxis durchaus bewährt; jedoch ist es in der Praxis nur schwierig und in manchen Fällen gar nicht möglich, eine ausreichende Anhaftung der aus den speziellen härtbaren Ausgangsmaterialien hergestellten Suprakonstruktionen an üblichen dentalen Abutments zu erreichen, wie sie im Zusammenhang mit dentalen Implantaten eingesetzt werden. Gemäß der WO 2008/037753 A2 wird deshalb auch mit speziell gestalteten Abutments gearbeitet, die Unterschneidungen besitzen und einen Formschluss mit komplementären provisorischen Suprakonstruktionen ermöglichen. Der Einsatz von Abutments ohne Unterschneidungen ist aber in der Praxis bevorzugt, da entsprechende Unterschneidungen durch inelastische Materialien (wie sie zum Beispiel im Anschluss an die Osseointegration bei Einsatz von permanenten Suprakonstruktionen verwendet werden), nicht ausgefüllt werden können. Zudem ist in der Praxis eine Verbindung zwischen einem solchen spezifischen Abutment mit Unterschneidungen und einer provisorischen Suprakonstruktion im manchen Fällen nicht ausreichend fest (wenn die provisorische Suprakonstruktion die Unterschneidung nicht vollständig ausfüllt) oder aber nahezu irreversibel (wenn nämlich die provisorische Suprakonstruktion fest in die Unterschneidungen einrastet). Daher werden Abutments nach wie vor in der dentalen Praxis typischerweise so gestaltet, dass sie einen sich zur Kaufläche (Okklusionsfläche) hin verjüngenden, z. B. konusförmigen Querschnitt aufweisen. Das Abweichen von dieser typischen Abutment-Gestalt wird in der dentalen Praxis als nicht erstrebenswert angesehen.

Das Dokument US 7,798,812 A1 trägt den Titel "Temporary dental prosthesis" und offenbart provisorische (temporäre) dentale Prothesen, die gegenüber Belastung nachgiebig sind und von außen applizierte Belastungen im Wesentlichen absorbieren. Als geeignete Materialien für derartige Provisorien werden wieder Silicone genannt, aber auch: Gummi (rubber), Nylon, Polyethylen, Copolymere, Kompomere, Elastomere, Kunststoffe (plastic), Teflon und andere biokompatible Materialien. Die in US 7,798,812 A1 offenbarten Suprakonstruktionen umfassen neben einem Prothesenkörper (prosthesis body) eine Grenzflächenstruktur (interface structure), die in ihrer Funktion einem üblichen Abutment entspricht. Prothesenkörper und Grenzflächenstruktur sind entweder integral miteinander verknüpft oder reversibel aneinander montiert, wobei insbesondere Schraubverbindungen eingesetzt werden. Die Lehre der US 7,798,812 A1 sieht keine Anbringung eines Prothesenkörpers auf einem typischen handelsüblichen Abutment (wie oben erläutert) vor. Weil die Systeme gemäß US 7,798,812 A1 den Einsatz spezifisch angepasster Grenzflächenstrukturen anstelle handelsüblicher Abutments erfordern, wird ihr Einsatz in manchen Fällen als nachteilig empfunden.

Eine primäre Aufgabe der vorliegenden Erfindung war es daher, eine provisorische Suprakonstruktion für ein dentales Implantat anzugeben, mit deren Hilfe die mit der Verwendung herkömmlicher provisorischer Suprakonstruktionen jeweils verbundenen Nachteile minimiert werden können, insbesondere die Nachteile, die bei Durchführung der technischen Lehren gemäß WO 2008/037753 A2 und US 7,798,812 A1 auftreten. Mit dieser primäre Aufgabe verknüpft waren dabei die weiteren Aufgaben, dentale Gemische oder dentale Mehrkomponentensysteme zur Herstellung des Materials einer provisorischen Suprakonstruktion für ein dentales Implantat oder eines Kerns für eine provisorische Suprakonstruktion für ein dentales Implantat anzugeben, einen entsprechenden provisorischen Zahnersatz anzugeben, ein Kit zur Herstellung einer Mehrzahl von provisorischen Suprakonstruktionen oder Kernen für provisorische Suprakonstruktionen anzugeben, die in Verfahren zur Beschleunigung der Osseointegration dentaler Implantate, vorzugsweise nach dem Prinzip des progressive bone loading, Einsatz finden können und entsprechende Verfahren zur provisorischen Versorgung eines dentalen Implantats anzugeben.

Hinsichtlich des anzugebenden dentalen Gemisches oder dentalen Mehrkomponentensystems zur Herstellung des Materials einer provisorischen Suprakonstruktion für ein dentales Implantat oder eines Kerns für eine provisorischen Suprakonstruktion für ein dentales Implantat wird diese Aufgabe überraschender Weise gelöst durch ein dentales Gemisch oder ein dentales Mehrkomponentensystem bestehend aus

(a) insgesamt 20,0 bis 99,9 Gewichtsprozent an ein, zwei oder mehr polymerisierbaren (Meth)Acrylaten

(b) insgesamt 0,1 bis 10,0 Gewichtsprozent an einem oder mehreren Katalysatoren und/oder Initiatoren für die Polymerisation,

(c) insgesamt 0,0 bis 60,0 Gewichtsprozent an einem oder mehreren anorganischen und/oder organischen Füllstoffen,

(d) insgesamt 0,0 bis 5,0 Gewichtsprozent an einem oder mehrerem Stabilisatoren, für die gilt: der oder die Stabilisatoren sind keine polymerisierbaren (Meth)Acrylate, sowie

(e) insgesamt 0,0 bis 50,0 Gewichtsprozent an optional einem oder mehreren weiteren Additiven,

wobei das dentale Gemisch bzw. das dentale Mehrkomponentensystem zu einem Produkt härtbar ist, wobei der durch (Polymerisations-)Härtung maximal erreichbare Druckmodul vorzugsweise zumindest 420 MPa oder weniger beträgt, zur Anwendung in einer therapeutischen Behandlung zur Beschleunigung der Osseointegration dentaler Implantate nach der Methode des progressive bone loading, wobei

- aus dem dentalen Gemisch bzw. dem Mehrkomponentensystem eine provisorische Suprakonstruktion für ein dentales Implantat oder ein Kern für eine provisorische Suprakonstruktion für ein dentales Implantat durch Polymerisationshärtung hergestellt wird

oder

- im Rahmen der Behandlung mehrere provisorische Suprakonstruktionen bestehend aus bzw. als Kernmaterial umfassend Materialien mit unterschiedlichen Druckmoduln hergestellt und in der Reihenfolge ansteigender Druckmoduln zeitlich nacheinander mit dem dentalen Implantat verbunden werden.

Der "durch Polymerisations- /Härtung maximal erreichbare Druckmodul" ist eine Materialeigenschaft. Das im Rahmen des vorliegenden Textes zu verwendende Verfahren zur Bestimmung dieser Eigenschaft wird weiter unten definiert. Die Herstellung eines geeigneten Prüfkörpers wird ebenfalls weiter unten erläutert.

Unter dem Begriff "Polymerisationshärtung" wird im vorliegenden Text das Verfestigen bzw. Härten eines Stoffes, eines Gemisches oder eines Mehrkomponentensystems durch Ausbildung von kovalenten Bindungen durch radikalische und/oder ionische Polymerisation oder Vernetzung verstanden, insbesondere durch radikalische Polymerisation. Nicht gemeint sind andere, insbesondere physikalische Verfahren, die zum Härten eines Gemisches führen, wie z.B. Sintern, Tempern oder Einfrieren.

Vom Begriff "(Meth)Acrylate" werden im vorliegenden Text Acrylate und Methacrylate umfasst.

Es war überraschend, dass dentale Gemische oder dentale Mehrkomponentensysteme, die aus einem, zwei oder mehr polymerisierbaren (Meth)Acrylaten sowie optional weiteren Bestandteilen bestehen, sämtliche Anforderungen an ein Ausgangsma-terial erfüllen, wie es im Rahmen der vorliegenden Erfindung angegeben werden sollte. Insbesondere sind nämlich die aus den genannten dentalen Gemischen oder dentalen Mehrkomponentensystemen herstellbaren Materialien und Produkte hervorragend zum Einsatz als provisorische Suprakonstruktion für ein dentales Implantat bzw. als Kern für eine solche provisorische Suprakonstruktion geeignet, wenn diese in einer therapeutischen Behandlung zur Beschleunigung der Osseointegration dentaler Implantate eingesetzt werden sollen, also insbesondere in einem Verfahren nach der Methode des progressive bone loading. Die aus den erfindungsgemäßen dentalen Gemischen oder dentalen Mehrkomponentensystemen durch Polymerisationshärtung herstellbaren Materialien vereinigen in sich sowohl die z. B. für das Verfahren des progressive bone loading gewünschte Eigenschaft eines niedrigen Druckmoduls mit einer hervorragenden Anhaftung an handelsüblichen, typischen, metallischen Abutments. Ausgehend von den erfindungsgemäßen dentalen Gemischen und dentalen Mehrkomponentensystemen lassen sich somit provisorische Suprakonstruktionen herstellen, die hervorragend an typischen Abutments anhaften und die gleichzeitig so weich sind, dass sie im Rahmen einer progressive bone loading-Therapie eingesetzt werden können, bei der sukzessive provisorische Suprakonstruktionen ansteigender Härte eingesetzt werden. Es sei insoweit auf die Ausführungen weiter unten und auf die grundlegenden Erläuterungen in der WO 2008/037753 A2 zu einer solchen Art der Therapie verwiesen.

Das erfindungsgemäße dentale Gemisch beziehungsweise das erfindungsgemäße dentale Mehrkomponentensystem dient zur Anwendung in einer spezifischen therapeutischen Behandlung zur Beschleunigung der Osseointegration dentaler Implantate nach der Methode des progressive bone loading, wobei aus dem dentalen Stoff bzw. dem dentalen Gemisch bzw. dem Mehrkomponentensystem eine provisorische Suprakonstruktion für ein dentales Implantat oder ein Kern für eine provisorische Suprakonstruktion für ein dentales Implantat durch Polymerisationshärtung hergestellt wird.

Dabei findet ein erfindungsgemäßes dentales Gemisch oder ein erfindungsgemäßes dentales Mehrkomponentensystem Anwendung in einer spezifischen therapeutischen Behandlung zur Beschleunigung der Osseointegration dentaler Implantate nach der Methode des progressive bone loading, wobei im Rahmen der Behandlung mehrere provisorische Suprakonstruktionen bestehend aus bzw. als Kernmaterial umfassend Materialien mit unterschiedlichen Druckmoduln hergestellt und in der Reihenfolge ansteigender Druckmoduln zeitlich nacheinander mit dem dentalen Implantat verbunden werden.

Wie bereits weiter oben ausgeführt, lassen sich "klassische" Verfahren des progressive bone loading (bei denen provisorische Suprakonstruktionen Einsatz finden, welche im Vergleich mit der zu restaurierenden dentalen Situation in ihrer Höhe verkürzt sind, so dass es in der Mundhöhle des Patienten im Normalfall nicht zu einer Okklusion mit den Antagonisten kommt) von zeitlich jüngeren Verfahren des progressive bone loading unterscheiden, in denen sukzessive Suprakonstruktionen ansteigender Härte eingesetzt werden, wobei diese regelmäßig Abmessungen besitzen, die denen der zu restaurierenden Situation entsprechen. Eine Okklusion mit dem Antagonisten in der Mundhöhle des Patienten ist daher möglich und sogar erwünscht. Die erfindungsgemäßen dentalen Gemische beziehungsweise dentalen Mehrkomponentensysteme sind somit insbesondere zur Anwendung in den letztgenannten Verfahren des progressive bone loading vorgesehen.

Die folgenden polymerisierbaren (Meth)Acrylate sind bevorzugte Bestandteile der Komponente (a) eines erfindungsgemäß anzuwendenden dentalen Gemisches oder dentalen Mehrkomponentensystems wobei das dentale Gemisch bzw. das dentale Mehrkomponentensystem zu einem Produkt härtbar ist, wobei der durch Härtung maximal erreichbare Druckmodul 420 MPa oder weniger beträgt:

1. Alkyl(meth)acrylate, deren Alkylgruppe zumindest 6 C-Atome besitzt.

2. Di(meth)acrylate des Polybutadiens, welche vorzugsweise herstellbar sind durch die Veresterung eines Hydroxy-funktionellen Polybutadiens mit (Meth)Acrylsäure (als hydroxy-funktionelle Polybutadiene sind beispielsweise Typen von "Poly BD" der Firma Cray Valley geeignet, z.B. Poly BD R-45HTLO Resin).

3. Alkyletherdi(meth)acrylate, deren Alkylgruppen unabhängig voneinander jeweils wenigstens 6 C-Atome besitzen.

4. Polyalkylether(mono/di/poly)(meth)acrylate, deren Alkylgruppen unabhängig voneinanderjeweils wenigstens 2 C-Atome besitzen und wobei die Anzahl der Etherfunktionen wenigstens 8 beträgt.

5. Aliphatische oder aromatische Urethandi(meth)acrylate, insbesondere:

5a. Aliphatische Urethandi(meth)acrylate, die herstellbar sind durch Umsetzung einer Diisocyanatkomponente, bevorzugt aus der Gruppe von Isophorondiisocyanat (IPDI), Hexamethylendiisocyanat (HDI), Cyclohexandiisocyanat, Dicyclopentadiendiisocyanat, hydriertem Methylendiphenyldiisocyanat (hydriertes MDI), besonders bevorzugt ist IPDI, mit einer eine (Meth)Acrylatfunktion tragenden Hydroxylkomponente, bevorzugt mit einem Hydroxyalkyl(meth)acrylat (beispielsweise Hydroxyethylmethacrylat (HEMA)) und/oder einem alkoxylierten Hydroxyalkyl(meth)acrylat, letzteres herstellbar aus einem molaren Überschuß an Ethylenoxid und/oder Propylenoxid und/oder Butylenoxid und/oder Tetrahydrofuran, etc. und (Meth)Acrylsäure, können Polymerisate mit hervorragenden flexiblen Eigenschaften bilden. Besonders bevorzugt ist die Reaktion des Diisocyanats mit ethoxyliertem und/oder propoxyliertem Hydroxyethylacrylat (HEA), Hydroxyethylmethacrylat (HEMA), Hydroxypropylacrylat (HPA), Hydroxypropylmethacrylat (HPMA), ganz besonders bevorzugt ist die Reaktion mit ethoxyliertem und/oder propoxyliertem Hydroxyethylmethacrylat (HEMA). Der Grad der Flexibilisierung des ausgehärteten Formstoffs kann durch die Stöchiometrie der Umsetzung zwischen dem cylischen Ether und der (Meth)Acrylsäure zum alkoxylierten Hydroxyalkyl(meth)acrylat als ein Edukt zur Isocyanat-Reaktion mit einem Diisocyanat maßgeschneidert eingestellt werden.
Aliphatische Urethandi(meth)acrylate sind kommerziell erhältliche Produkte oder können nach bekannten Methoden, die ein Fachmann beherrscht, synthetisiert werden.
5b. Aliphatische oder aromatische Urethandi(meth)acrylate, die herstellbar sind durch Umsetzung einer Diisocyanatkomponente aus der Gruppe der aromatischen und aliphatischen Diisocyanate mit einer eine (Meth)acrylatfunktion tragenden Hydroxylkomponente basierend auf Polybutadiendiolen.
Insbesondere geeignet sind aliphatische oder aromatische Urethandi(meth)acrylate, die herstellbar sind durch Umsetzung einer Diisocyanatkomponente aus der Gruppe der aromatischen und aliphatischen Diisocyanate mit $\alpha$-(Hydroxy)-$\omega$-[(meth)acryl]-polybutadien, welches herstellbar ist durch die Veresterung eines Hydroxyfunktionellen Polybutadiens (z.B. "Poly BD R-45HTLO Resin" von Cray Valley) mit einem Äquivalent (Meth)Acrylsäure.

6. Alkoxylierte Bisphenoldi(meth)acrylate, deren Alkylgruppen unabhängig voneinander jeweils wenigstens 2 C-Atome besitzen und wobei die Gesamtzahl der Etherfunktionen wenigstens 10 beträgt.

Kombinationen (Gemische) von (Meth)Acrylaten wie unter den vorstehenden Punkten 1 bis 6 definiert miteinander und/oder mit weiteren (Meth)Acrylaten sind in manchen Fällen vorteilhaft, insbesondere Kombinationen von vorstehend genannten (Meth)Acrylaten der Gruppen 1 - 6 mit Polyethylenglykoldimethacrylat, z. B. von aliphatischem Urethandi(meth)acrylat (siehe oben Gruppen 5a und 5b) mit Polyethylenglykoldimethacrylat (n=9) (siehe oben Gruppe 3). Der Fachmann wird anhand einfacher Vorversuche ermitteln, ob ein entsprechendes Gemisch nach Polymerisationshärtung den relevanten Druckmodul von weniger als 420 MPa besitzt und zur Anwendung in einer therapeutischen Behandlung zur Beschleunigung der Osseointegration dentaler Implantate, insbesondere nach der Methode des progressive bone loading, geeignet ist (dazu muss es in fester Phase vorliegen). Im Bedarfsfall wird der Fachmann durch Variation weiterer Bestandteile die gewünschten Eigenschaften einstellen.
Bevorzugte erfindungsgemäß anzuwendende dentale Gemische umfassen in Komponente (a) vorzugsweise ein (Meth)Acrylat wie es vorstehend gemäß den Punkten 1 bis 6 definiert ist. Bevorzugt einzusetzende bzw. in Kombination bevorzugt einzusetzende (Meth)Acrylate sind nachfolgend angegeben:

Poly-Alkylether-Tri(meth)-acrylate, Poly-Alkylether-Poly(meth)acrylate, Poly-Alkylether-Alkoxy-Di(meth)acrylate, Poly-Alkylether-Alkoxy-Tri(meth)-acrylate, Poly-Alkylether-Alkoxy-Poly(meth)acrylate, Poly-Aliphatische-Urethandi(metha)crylate, Poly-Aliphatische-Urethan(metha)crylate, Polyethylenglykol-Di(meth)acrylate, Polypropylenglykol-Di(meth)acrylate, Polyisopropylenglykol-Di(meth)acrylate, Polyisobutylenglykol-Di(meth)acrylate, Bisphenol-A-Alkoxylat-Di(meth)acry-late, Bisphenol-F-Alkoxylat-Di(meth)acrylate, Bisphenol-B-Alkoxylat-Di(meth)acrylate, Ethoxylierte Bisphenol-A-Di(meth)acrylate, Ethoxylierte Bisphenol-F-Di(meth)acrylate, Ethoxylierte Bisphenol-B-Di(meth)acrylate, Propoxylierte Bisphenol-A-Di(meth)acrylate, Propoxylierte Bisphenol-F-Di(meth)acrylate, Propoxylierte Bisphenol-B-Di(meth)acrylate und weitere Alkoxylierte Bisphenol-Derivate Di(meth)acrylate.

Bevorzugte (Meth)Acrylate zum Einsatz in Komponente (a) eines erfindungsgemäß anzuwendenden dentalen Gemisches oder dentalen Mehrkomponentensystems sind u.a. aufgeführt in I.) Lackrohstoff-Tabellen; Erich Karsten; 10. Auflage; Vincentz Verlag Hannover; 2000; II.) Polymer Handbook; 4th edition; Editors: J.Brandrup, E.H. Immergut & E.A. Grulke; Wiley Verlag; 1999 und III.) Chemistry & Technology of UV&EB Formulation for Coatings, Inks & Paints:

Volume III -Prepolymers & Reactive Dilutents; Editor: G. Webster; SITA Technology Ltd. London; publiziert von John Wiley & Sons Ltd., London, 1997. Kommerziell verfügbare Verbindungen sind u.a. von der Firma Atofina bzw. deren Subsidiär-Firmen Sartomer und Cray Valley erhältlich. Beispiele werden nachfolgend aufgeführt:

2-(2-Ethoxyethoxy) Ethyl Acrylat ((EOEOEA) SR256), 2-Phenoxyethyl Acrylat ((PEA) SR339C), Caprolactone Acrylat (SR495), Cyclic Trimethylopropane Formal Acrylat ((CTFA) SR531), Ethoxylated$_4$ Nonyl Phenol Acrylat (SR504), Isobornyl Acrylat ((IBOA) SR506D), Isodecyl Acrylat ((IDA) SR395), Lauryl Acrylat (SR335), Octyl Decyl Acrylat ((ODA) SR484), Stearyl Acrylat (SR257C), Tetrahydrofurfuryl Acrylat ((THFA) SR285), Tridecyl Acrylat (SR489), Alkoxylated Diacrylat (SR802), Alkoxylated Hexanediol Diacrylat (CD561), Esterdiol Diacrylat (SR606A), Ethoxylated$_{10}$ Bisphenol A Diacrylat (SR602), Polyethylene Glycol 400 Diacrylat ((PEG400DA) SR344), Polyethylene Glycol 600 Diacrylat ((PEG600DA) SR610), Ethoxylated$_{15}$ Trimethylolpropane Triacrylat (CN435), Ethoxylated$_{20}$ Trimethylolpropane Triacrylat (SR415), Ethoxylated$_9$ Trimethylolpropane Triacrylat (SR502), Highly propoxylated Glycerol Triacrylat (SR9021), Modified Pentaerythritol Triacrylat (SR444), 2-Phenoxyethyl Methacrylat (SR340), Ethoxylated$_{10}$ Hydroxyethyl Methacrylat (CD572), Isobornyl Methacrylat (SR423A), Lauryl Methacrylat (SR313E), Methoxy Polyethylene Glycol 350 Monomethacrylat (CD550), Methoxy Polyethylene Glycol 550 Monomethacrylat (CD552), Polypropylene Glycol Monomethacrylat (SR604), Stearyl Methacrylat (SR324D), Tetrahydrofurfuryl Methacrylat ((THFMA) SR203), Ethoxylated$_{10}$ Bisphenol A Dimethacrylat (SR480), Polyethylene Glycol 600 Dimethacrylat ((PEG600DMA) SR252), Polybutadiene, dimethacrylat (CN301), Difunctional Polyester Acrylate (CN UVP210), Hexafunctional Polyester Acrylate (CN293), Polyester Acrylate (CN203), Polyester Acrylate (SYNOCURE AC1007), etc.

Bevorzugte erfindungsgemäß anzuwendende Gemische oder Mehrkomponentensysteme wie vorstehend oder nachfolgend spezifiziert umfassen in oder als Komponente (a) ein oder mehrere (Meth)acrylate ausgewählt aus der Gruppe bestehend aus:

1. Alkyl(meth)acrylate, deren Alkylgruppe zumindest 6 C-Atome besitzt;

2. Di(meth)acrylate des Polybutadiens;

3. Alkyletherdi(meth)acrylate, deren Alkylgruppen unabhängig voneinander jeweils wenigstens 6 C-Atome besitzen;

4. Polyalkylether(mono/di/poly)(meth)acrylate, deren Alkylgruppen unabhängig voneinander jeweils wenigstens 2 C-Atome besitzen und wobei die Anzahl der Etherfunktionen wenigstens 8 beträgt;

5. Aliphatische oder aromatische Urethandi(meth)acrylate;

6. Alkoxylierte Bisphenoldi(meth)acrylate, deren Alkylgruppen unabhängig voneinander jeweils wenigstens 2 C-Atome besitzen und wobei die Gesamtzahl der Etherfunktionen wenigstens 10 beträgt.

Ein erfindungsgemäß anzuwendendes Gemisch oder Mehrkomponentensystem umfasst vorzugsweise in oder als Komponente (c) ein oder mehrere partikuläre anorganische und/oder organische Füllstoffe, die vorzugsweise ausgewählt sind aus der Gruppe bestehend aus Siliciumdioxid, Metalloxiden und Poly(meth)acrylaten.

Ganz besonders bevorzugt ist der Einsatz von oberflächenmodifizierten oder nicht-oberflächenmodifizierten $SiO_2$-Partikeln eines mittleren Teilchendurchmessers von unter 200 nm in oder als Komponente (c). Neben diesen $SiO_2$-Partikeln können optional weitere partikuläre anorganische oder organische Füllstoffe vorhanden sein.

Die oberflächenmodifizierten oder nicht-oberflächenmodifizierten $SiO_2$-Partikel eines mittleren Teilchendurchmessers von unter 200 nm des Bestandteils (c) sind vorzugsweise pyrogenes $SiO_2$ wie es etwa unter dem Namen AEROSIL® (Evonik) angeboten wird. Diese Teilchen beeinflussen sowohl die Härte des polymerisationsgehärteten dentalen Gemisches oder Mehrkomponentensystems als auch die Konsistenz des dentalen Gemisches oder Mehrkomponentensystems selbst und sind vorzugsweise oberflächlich modifiziert. Die in Bestandteil (c) optional weiter enthaltenen partikulären anorganischen oder organischen Füllstoffe sind vorzugsweise siliciumhaltig und umfassen z.B. gemahlenen Quarz, beispielsweise eines mittleren Teilchendurchmessers von ca. 10 μm.

Als partikuläre anorganische und/oder organische Füllstoffe sind grundsätzlich alle inerten Materialien geeignet. Die partikulären Füllstoffe haben Einfluss auf den Druckmodul des erfindungsgemäßen dentalen Gemisches oder Mehrkomponentensystems, welches als Ausgangsmaterial für die Herstellung von Suprakonstruktionen bzw. Kerne dient. Beispiele für anorganische Füllstoffe sind Gläser, insbesondere Dentalgläser, Siliciumdioxid (z.B. als Quarze, Cristobalite, pyrogenes $SiO_2$ wie etwa das unter dem Namen AEROSIL® (Evonik) angebotene und/oder nach einem Sol-Gel-Verfahren hergestelltes $SiO_2$), Beispiele für organische Füllstoffe sind Poly(meth)acrylate. Die Füllstoffe werden während der Polymerisationshärtung der reaktiven Bestandteile insbesondere der Komponente (a) des erfindungsgemäßen dentalen Gemisches oder Mehrkomponentensystems in die Matrix eingebaut und verleihen dem gehärtetem Produkt (beispielsweise der unmittelbar einsetzbaren dentalen Suprakonstruktion bzw. dem Kern) Festigkeit.

Vorzugsweise ist das erfindungsgemäß anzuwendende dentale Gemisch oder das erfindungsgemäß anzuwendende dentale Mehrkomponentensystem ebenso wie das daraus resultierende Produkt (insbesondere eine erfindungsgemäße dentale Suprakonstruktion oder ein erfindungsgemäßer Kern) gefärbt, insbesondere um seine Farbe an eine Farbe natürlicher Zähne anzupassen. Beispiele für farbgebende Bestandteile sind z.B. ein oder mehrere anorganische oder organische Füllstoffe des Bestandteils (c) (z.B. zahnfarbene Poly(meth)acrylat-Partikel, Metalloxide und/oder gemahlener Quarz). Es können jedoch auch zusätzliche farbgebende Bestandteile als bzw. in der optionalen Komponente (e) vorgesehen sein, beispielsweise natürliche Farbstoffe oder synthetische Farbstoffe wie Azo-, Triarylmethan-, Chinophthalon-, Xanthen- und Indigofarbstoffe sowie Anthrachinon-, Nitroso- und Phthalocyaninfarbstoffe.

Zusätzliche polymerisierbare Verbindungen können als bzw. in der optionalen Komponente (e) vorgesehen sein.

Partikuläre Füllstoffe, welche einen festen anorganischen oder organischen Kern besitzen und polymerisierbare (Meth)Acrylat-Gruppen an ihrer Oberfläche aufweisen, werden insbesondere bei quantitativen Mengenangaben nicht zu den polymerisierbaren (Meth)Acrylaten der Komponente (a) gezählt, sondern zu Komponente (c). In allen übrigen Fällen werden Bestandteile, welche technisch und begrifflich zwei oder mehr Komponenten zugeordnet werden können, für die Zwecke quantitativer Angaben der früher genannten Komponente zugeordnet (also (a) vor (b) vor (c) vor (d) vor (e) usw.).

Besonders bevorzugte erfindungsgemäß anzuwendende dentale Gemische oder erfindungsgemäße dentale Mehrkomponentensysteme, wie sie zur Anwendung in den oben genannten Therapieverfahren vorgesehen sind, sind Gemische, welche chemisch härtbar und/oder lichthärtbar sind. Vorzugsweise sind erfindungsgemäß anzuwendende dentale Gemische oder dentale Mehrkomponentensysteme sowohl chemisch härtbar als auch lichthärtbar. Sie ermöglichen somit eine Härtung nach dem dual-cure-Verfahren. Zum Beispiel lassen sich die dentalen Gemische anfangs mit Licht vorhärten, um eine gewisse Grundfestigkeit zu erhalten, und härten anschließend chemisch nach. Bevorzugt für das dual-cure-Verfahren eignen sich Mehrkomponenten-Systeme mit zwei oder mehr Komponenten (umfassend voneinander verschiedene dentale Gemische), die zu Beginn der Härtung miteinander zu einem dentalen Gemisch vermischt werden.

Erfindungsgemäß anzuwendende dentale Zweikomponentensysteme weisen eine erste und eine zweite Komponente auf, die vorzugsweise im Volumenverhältnis von 10:1 bis 1:10, bevorzugt 4:1 bis 1:4, besonders bevorzugt 2:1 bis 1:2 und insbesondere 1:1 zu einem dentalen Gemisch anzumischen sind. Bevorzugt findet die Durchmischung in einer statischen Mischvorrichtung statt. Besonders bevorzugt sind die erste und die zweite Komponente so aneinander angepasst, dass nach dem Mischen die Vernetzung innerhalb eines Zeitraums von 30 s bis 10 min. abgeschlossen ist.

Bei lichthärtenden Systemen umfasst die Komponente (b) eines erfindungsgemäß anzuwendenden dentalen Gemisches einen Lichthärtungsinitiator. Beispiele für einen Lichthärtungsinitiator schließen Katalysatoren ein, die nur photosensibilisierend wirken sowie Kombinationen aus Sensibilisator und Beschleuniger.

Beispiele für Photosensibitisatoren sind alpha-Diketone, Benzoinalkylether, Thioxanthone, Benzophenone, Acylphosphinoxide, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2. Weitere geeignete Initiatoren sowie Initiatorkombinationen sind in der DE 601 16 142 beschrieben.

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung eines erfindungsgemäßen härtbaren Gemisches oder Mehrkomponentensystems bewirken können.

Das Absorptionsmaximum von Campherchinon (CC) liegt bei ca. 470 nm und somit im Bereich des blauen Lichts. Campherchinon (CC) zählt zu den $PI_2$-Initiatoren und wird regelmäßig zusammen mit einem Coinitiator eingesetzt. Vorzugsweise enthält ein erfindungsgemäßes härtbares Gemisch oder Mehrkomponentensystem die Kombination eines alpha-Diketons und eines aromatischen tertiären Amins, bevorzugt ist die Kombination von Campherchinon (CC) und Ethyl-*p-N,N*-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in erfindungsgemäßen härtbaren Gemischen wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600

EP 2 481 391 B1

29 481 T2, EP 0 980 682 B1, EP 0 948 955 B1, EP 1 236 459 B1 und EP 0 173 567 A2 verwiesen.

Die in diesen Druckschriften angegebenen Phosphinoxide eignen sich besonders allein oder in Kombination mit dem System "alpha-Diketon/Amin" als Photopolymerisationsinitiatorsystem in den erfindungsgemäßen Gemischen oder Mehrkomponentensystemen.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden.

Weitere geeignete Photoinitiatoren (Lichthärtungsinitiatoren) sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben.

Bei chemisch härtenden Systemen umfasst die Komponente (b) eines erfindungsgemäß anzuwendenden Gemisches einen Initiator für eine chemische Aushärtung. Dem Fachmann sind diverse Initiatoren für eine chemische Aushärtung bekannt. Es sei insoweit exemplarisch auf die EP 1 720 506 verwiesen. Bevorzugte Initiatoren zur chemischen Härtung sind Benzoylperoxid, Lauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Die Peroxide und die Amine werden dabei auf zwei unterschiedliche Komponenten eines Dentalmaterials aufgeteilt, die jeweils für sich bereits ein dentales Gemisch darstellen. Beim Mischen der aminhaltigen Komponente (sogenannte Basispaste) mit der peroxidhaltigen Komponente (sogenannte Initiator- oder Katalysatorpaste) zu einem dentalen (Folge-) Gemisch wird durch die Reaktion von Amin und Peroxid (Redoxreaktion) die radikalische Reaktion initiiert.

Dualhärtende Systeme umfassen eine Kombination aus Photoinitiatoren und Initiatoren zur chemischen Aushärtung. Beispielsweise kann eine (als erste Komponente eingesetzte) Basispaste zusätzlich einen Photoinitiator enthalten, so dass die Basispaste entweder allein als (als zweite Komponente einzusetzende) lichthärtender oder zusammen mit einer Initiatorpaste als licht- und selbsthärtender Dentalwerkstoff (dentalen Gemisch) eingesetzt werden kann. Neben den oxidativ wirksamen organischen Peroxidverbindungen können als Redoxsysteme auch Barbitursäuren bzw. Barbitursäurederivate sowie Malonylsulfamide verwendet werden. Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023.

Geeignete Malonylsulfamide sind in der EP 0 059 451 beschrieben. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

Anstelle der Barbitursäure/-derivate können auch Salze C-H aktiver Verbindungen eingesetzt werden. EP 1 872 767, EP 2 070 506, DE 11 2006 001 049, US 7,214,726, EP 2 070 935 sowie WO 2007/131725 beschreiben geeignete Systeme.

Besonders bevorzugte erfindungsgemäß anzuwendende dentale Gemische oder erfindungsgemäße dentale Mehrkomponentensysteme, wie sie zur Anwendung in den oben genannten Therapieverfahren vorgesehen sind, umfassen

(a) insgesamt 44,9 bis 99,9 Gewichtsprozent an polymerisierbaren (Meth)Acrylaten,

(b) insgesamt 0,1 bis 5,0 Gewichtsprozent an Katalysatoren und Initiatoren für die Polymerisation,

(c) insgesamt 0,0 bis 55,0 Gewichtsprozent an anorganischen und organischen Füllstoffen,

(d) insgesamt 0,0 bis 2,0 Gewichtsprozent Stabilisatoren, für die gilt: der oder die Stabilisatoren sind keine polymerisierbaren (Meth)Acrylate, sowie

(e) insgesamt 0,0 bis 5,0 Gewichtsprozent an weiteren Additiven.

Die vorliegende Erfindung betrifft auch eine provisorische Suprakonstruktion für ein dentales Implantat sowie einen Kern für eine provisorische Suprakonstruktion für ein dentales Implantat, herstellbar durch Polymerisationshärtung eines erfindungsgemäß anzuwendenden dentalen Gemisches oder eines erfindungsgemäß anzuwendenden dentalen Mehrkomponentensystems.

Entsprechend den Materialeigenschaften der erfindungsgemäß anzuwendenden dentalen Gemische oder Mehrkomponentensysteme, die oben bereits erläutert wurden, besitzt das Material der provisorische Suprakonstruktion beziehungsweise das Material des Kerns für eine provisorische Suprakonstruktion erfindungsgemäß zumindest vorzugsweise einen Druckmodul von weniger als 420 MPa, ist also bei Druckbelastung besonders weich. Aufgrund dieses niedrigen Druckmoduls ist eine erfindungsgemäße provisorische Suprakonstruktion beziehungsweise ein entsprechender Kern besonders gut zum Einsatz in den bevorzugten progressive bone loading-Verfahren geeignet, bei denen sukzessive mit Materialien ansteigender Härte gearbeitet wird. Bei Einsatz erfindungsgemäßer provisorischer Suprakonstruktionen bzw. Kerne ist es im Unterschied zu den "klassischen" progressive bone loading-Verfahren nicht mehr erforderlich, die Abmessungen der provisorischen Suprakonstruktionen im Vergleich mit den Abmessungen des zu restaurierenden Zahnes zu verringern. Aufgrund der erfindungsgemäßen Wahl des geeigneten Materials (Suprakonstruktion oder Kern auf Basis von (Meth)Acrylaten) wird zudem eine hervorragende Anhaftung an einem üblichen metallischen Abutment gewährleistet, die gemäß den Verfahren des Standes der Technik nicht erreicht wurde.

Gemäß einer ersten alternativen Ausgestaltung einer erfindungsgemäßen Suprakonstruktion besteht diese aus einem Kern und einer Kappe für den Kern, wobei die Außenfläche des Kerns teilweise frei liegt, so dass der Kern bei Druckbelastung zumindest abschnittsweise unabhängig von der Kappe deformierbar ist, wobei das Material der Kappe vorzugsweise einen höheren Druckmodul aufweist als das Material des Kerns.

Die Kappe dient hierbei vorzugsweise dazu, eine Oberfläche mit gegenüber der Oberfläche (Außenfläche) des Kerns verbesserten Kaueigenschaften (insbesondere einer erhöhten Abrasionsfestigkeit) bereitzustellen. Material, Dicke und Form der Kappe sind so ausgewählt und an den Kern angepasst, dass die Fähigkeit des Kerns, Kaukräfte zu absorbieren und reduziert auf das Implantat zu übertragen, erhalten bleibt. Die Kappe ist vorzugsweise so beschaffen, dass sie nur einen Teil der äußeren Oberfläche (Außenfläche) des Kerns bedeckt, wobei dieser Teil die Kaufläche umfasst. Die Kappe besteht vorzugsweise aus Keramik oder aus einem üblichen provisorischen Kronenmaterial vergleichsweise hoher Härte (z.B. Kunststoffen oder Kompositen auf Kunststoffbasis, etwa auf Basis von (Meth)Acrylathaltigen Verbindungen wie Polymethylmethacrylat (PMMA), Bisphenol-A-Glycidylmethacrylat (Bis-GMA), Urethandimethacrylat (UDMA) oder Triethylenglycoldimethacrylat (TEGDMA); siehe auch die obigen Ausführungen zu herkömmlichen dentalen Prothesenkunststoffen. Die Kappe wird auf einen zugeordneten Kern beispielsweise aufgeklebt oder unter Ausbildung eines Klemmsitzes (gegebenenfalls mit Formschluss) auf diesem angeordnet, um die provisorische Suprakonstruktion herzustellen.

Gemäß einer zweiten alternativen Ausgestaltung einer erfindungsgemäßen provisorischen Suprakonstruktion besitzt diese keine Kappe der vorstehend geschilderten Art. Vorzugsweise ist eine erfindungsgemäße provisorische Suprakonstruktion dann herstellbar durch eine einzelne Polymerisationshärtung eines einzelnen erfindungsgemäß anzuwendenden dentalen Gemisches.

Wie bereits erläutert, bezeichnet der Begriff "provisorische Suprakonstruktion" eine Suprakonstruktion, die von vornherein dazu bestimmt ist, zu einem späteren Zeitpunkt durch eine andere Suprakonstruktion ersetzt zu werden. In der Regel ist eine solche provisorische Suprakonstruktion dazu vorgesehen, einige Tage, Wochen oder Monate im Mund zu verbleiben, bis die Versorgung mit einer endgültigen Suprakonstruktion erfolgt. Die erfindungsgemäße provisorische Suprakonstruktion kann direkt nach der Implantation (Sofortbelastung) oder nach einer verkürzten oder herkömmlichen Einheilzeit (z. B. 3 bis 6 Monate) über ein Abutment oder direkt mit dem dentalen Implantat verbunden werden, vorzugsweise so, dass die provisorische Suprakonstruktion in Okklusion mit dem Gegenkiefer steht. Die provisorische Suprakonstruktion gewährleistet so, dass Kaukräfte aufgenommen und aufgrund der Dehnbarkeit und Flexion der provisorischen Suprakonstruktion reduziert auf das Implantat weitergegeben werden. Vorzugsweise stellen während der Einheilphase aufeinander abgestimmte provisorische Suprakonstruktionen ansteigender Härte sicher, dass die Kontaktfläche zwischen Implantat und Kieferknochen zunehmenden Belastungen unterworfen wird. Die Patienten müssen sich nicht auf Brei beschränken, sondern können ihre Nahrung je nach der funktionellen Ess- und Kaufunktion ihrer provisorischen Suprakonstruktion selbst bestimmen. Der Einsatz einer erfindungsgemäßen provisorischen Suprakonstruktion ist zudem aus ästhetischen Gründen vorteilhaft, da keine Zahnlücke auftritt. Für die erfindungsgemäßen Suprakonstruktionen und Kerne ist ihr Druckmodul von entscheidender Bedeutung, wie vorstehend bereits ausgeführt wurde.

Bevorzugt ist eine erfindungsgemäße provisorische Suprakonstruktion oder ein erfindungsgemäßer Kern für eine provisorische Suprakonstruktion, wobei die provisorische Suprakonstruktion eine provisorische Krone ist. Unter "Krone" wird im Rahmen des vorliegenden Textes ein Element verstanden, das zum Ersatz eines einzelnen Zahnes bestimmt ist und dessen Retention auf einem anderen Element im Wesentlichen auf einem Formschluss oder Kraftschluss zwischen (zumindest einem Teil) seiner inneren Oberfläche und einer äußeren Oberfläche des anderen Elements beruht. Vorzugsweise ist eine Krone der natürlichen Zahngeometrie angenähert. Hierdurch ist eine kontrollierte Krafteinleitung besonders gut möglich. Eine erfindungsgemäße provisorische Suprakonstruktion kann jedoch auch ein provisorischer Brückenpfeiler sein.

Die Erfindung betrifft auch einen provisorischen Zahnersatz umfassend

- eine erfindungsgemäße provisorische Suprakonstruktion (die bzw. deren Material also herstellbar ist durch Polymerisationshärtung eines erfindungsgemäß anzuwendenden dentalen Gemisches),

- ein dentales Implantat

und

- ein vorzugsweise metallisches Abutment, das zwischen provisorischer Suprakonstruktion und dentalem Implantat angeordnet ist und deren Verbindung gewährleistet

sowie optional

- einen Haftvermittler, der zwischen provisorischer Suprakonstruktion und Abutment angeordnet ist.

Ein dentales Implantat besteht vorzugsweise aus Titan und ein bevorzugtes Abutment besteht aus Metall, wobei Titan besonders häufig eingesetzt wird.

In bevorzugten erfindungsgemäßen provisorischen Zahnersätzen (dentalen Provisorien) sind die provisorische Suprakonstruktion und das Abutment ohne Formschluss in Kraftrichtung entlang der Längsachse des dentalen Implantats vom Kieferknochen weg, in den das Implantat eingesetzt oder einzusetzen ist, verbunden.

Eine Verbindung ohne solchen Formschluss wird beispielsweise bei den bereits weiter oben erläuterten Abutments erreicht, die so gestaltet sind, dass sie einen sich zur Kaufläche (Okklusionsfläche) hin verjüngenden, z. B. konusförmigen Querschnitt besitzen. Besonders bevorzugte erfindungsgemäße provisorische Zahnersätze umfassen ein metallisches Abutment, welches einen sich zur Kaufläche (Okklusionsfläche) hin verjüngenden Querschnitt besitzt, so dass die provisorische Suprakonstruktion und das Abutment verbunden sind ohne Formschluss in Kraftrichtung entlang der Längsachse des dentalen Implantats vom Kieferknochen weg, in den das Implantat eingesetzt oder einzusetzen ist. Besonders bevorzugt ist es, wenn das eingesetzte metallische Abutment einen konusförmigen Querschnitt besitzt. Erfindungsgemäße provisorische Zahnersätze umfassen nicht in jedem Falle einen Haftvermittler zwischen provisorischer Suprakonstruktion und Abutment. Ein Haftvermittler ist vielmehr aufgrund der hervorragenden Eigenhaftungen der provisorischen Suprakonstruktion am Abutment im Regelfall entbehrlich.

Sofern zur Verbesserung der Anhaftung der provisorischen Suprakonstruktion an dem Abutment ein Haftvermittler eingesetzt wird, wird dieser vorzugsweise ausgewählt aus der Gruppe der eugenolfreien Zemente auf Zinkoxid-Basis (beispielsweise Provicol (VOCO), RelyX Temp E (3M Espe)) oder der (meth)acrylatbasierenden temporären Befestigungskomposite (beispielsweise Telio CS Link (Ivoclar Vivadent) und implantlink® semi (Detax)).

Bevorzugte Stabilisatoren (Inhibitoren) zum Einsatz in erfindungsgemäß anzuwendenden dentalen Gemischen oder Mehrkomponentensystemen werden üblicherweise zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität des lichthärtbaren dentalen Gemisches bzw. des Mehrkomponentensystems. Gängige Stabilisatoren (Inhibitoren) sind Phenolderivate wie Hydrochinonmonomethylether (HQME) oder 2,6-Di-tert.butyl-4-methylphenol (BHT). Weitere Stabilisatoren (Inhibitoren) wie tert.-Butylhydroxyanisol (BHA), 2,2 Diphenyl-1-picryl-hydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben. Alternative Stabilisatoren (Inhibitoren) sind in der DE 101 19 831 A1 und in der EP 1 563 821 A1 angegeben.

Bevorzugte Additive zum Einsatz in erfindungsgemäß anzuwendenden dentalen Gemischen oder Mehrkomponentensystemen sind beispielsweise UV-Absorber, die beispielsweise durch ihre konjugierten Doppelbindungssytemen und aromatischen Ringe befähigt sind, UV Strahlung zu absorbieren. Beispiele bevorzugter UV-Absorber sind 2-Hydroxy-4-methoxybenzophenon, Salicylsäurephenylester, 3-(2'-Hydroxy-5'-methylphenyl)-benzotriazol und Diethyl-2,5-dihydroxy-terephthalat.

Die vorliegende Erfindung betrifft auch ein Kit zur Herstellung einer Mehrzahl von provisorischen Suprakonstruktionen für ein dentales Implantat oder einer Mehrzahl von Kernen für provisorische Suprakonstruktionen für ein dentales Implantat, umfassend

- eine entsprechende Anzahl erfindungsgemäß anzuwendender dentaler Gemische oder dentaler Mehrkomponentensysteme wie vorstehend definiert, wobei jedes der dentalen Gemische oder Mehrkomponentensysteme zu einem Produkt härtbar ist, wobei der durch (Polymerisations-)Härtung maximal erreichbare Druckmodul somit vorzugsweise jeweils 420 MPa oder weniger beträgt und wobei der Druckmodul der Produkte bei gleichen Härtungsbedingungen unterschiedlich ist

oder

- eine entsprechende Anzahl von Rohlingen aus einem gehärteten bzw. durch Härtung herstellbar aus einem, erfindungsgemäß anzuwendenden dentalen Gemisch wie vorstehend definiert, wobei der Druckmodul des Materials jedes der Rohlinge unterschiedlich ist und zumindest vorzugsweise jeweils 420 MPa oder weniger beträgt,

sowie

- optional ein oder mehrere weitere Bestandteile ausgewählt aus der Gruppe bestehend aus Haftvermittler, Abutments, Implantate, Formwerkzeuge (Formen), Abformmaterialien.

Unter einem "Rohling" ist hierbei ein Vorläufer einer erfindungsgemäßen provisorischen Suprakonstruktion oder eines erfindungsgemäßen Kerns zu verstehen, der im Allgemeinen nicht in unveränderter Form mit einem Implantat bzw. einem Abutment verbunden werden kann, sondern dazu bestimmt ist, hinsichtlich seiner Form an eine bestimmte Patientensituation angepasst zu werden, beispielsweise durch Entfernen (z.B. Abschneiden, Abschleifen oder ein sonstiges abtragendes formgebendes Verfahren) von Überschüssen.

Ein erfindungsgemäßes Kit umfasst in manchen bevorzugten Ausgestaltungen auch Materialien zur Herstellung einer Form durch Abformung und/oder Mittel zur Herstellung einer isolierenden Schicht auf einem Abutment zum Verhindern oder Reduzieren der Anhaftung eines aufgepressten, gehärteten Produkts und/oder Gerüst-Bausteine zum Aufbau einer abnehmbaren dentalen Modulation und/oder einer provisorischen Suprakonstruktion und/oder ein oder mehrere Materialien zur Herstellung einer abnehmbaren dentalen Modulation.

Bevorzugt ist ein erfindungsgemäßes Kit zum Herstellen mehrerer (z.B. 2, 3 oder mehr) erfindungsgemäßer provisorischer Suprakonstruktionen oder Kerne, deren Materialien sich hinsichtlich ihrer Druckmoduln unterscheiden. Die herzustellenden provisorischen Suprakonstruktionen bzw. Kerne sind regelmäßig dazu bestimmt, in der Reihenfolge ansteigender Druckmoduln zeitlich nacheinander mit dem dentalen Implantat verbunden zu werden. Besonders bevorzugt ist ein erfindungsgemäßes Kit zum Herstellen von 3 oder mehr provisorischen Suprakonstruktionen oder Kernen, wobei jeweils eine oder mehrere provisorische Suprakonstruktionen bzw. Kerne aus Materialien mit Druckmoduln in den Bereichen (i) < 25 MPa, (ii) 25 MPa bis < 100 MPa und (iii) 100 MPa bis 420 MPa bestehen.

Ein besonders bevorzugtes erfindungsgemäßes Kit umfasst

- ein erstes erfindungsgemäß anzuwendendes dentales Gemisch oder ein erstes erfindungsgemäß anzuwendendes dentales Mehrkomponentensystem wie vorstehend definiert, wobei das erste dentale Gemisch bzw. das erste dentale Mehrkomponentensystem zu einem Produkt härtbar ist, wobei der durch Härtung (Polymerisationshärtung) maximal erreichbare Druckmodul im Bereich von 25 MPa bis kleiner 100 MPa liegt,

- ein zweites erfindungsgemäß anzuwendendes dentales Gemisch oder ein zweites erfindungsgemäß anzuwendendes dentales Mehrkomponentensystem wie vorstehend definiert, wobei das zweite dentale Gemisch bzw. das zweite dentale Mehrkomponentensystem zu einem Produkt härtbar ist, wobei der durch Härtung (Polymerisationshärtung) maximal erreichbare Druckmodul im Bereich von 100 MPa bis 420 MPa liegt,

- optional ein drittes dentales Gemisch oder ein drittes dentales Mehrkomponentensystem, vorzugsweise ein erfindungsgemäß anzuwendendes drittes dentales Gemisch oder ein erfindungsgemäß anzuwendendes drittes dentales Mehrkomponentensystem wie vorstehend definiert, wobei das dritte dentale Gemisch bzw. das dritte dentale Mehrkomponentensystem zu einem Produkt härtbar ist, wobei der durch Härtung (Polymerisationshärtung) maximal erreichbare Druckmodul kleiner ist als 25 MPa,

- optional ein weiteres dentales Gemisch oder ein weiteres dentales Mehrkomponentensystem, wobei das weitere dentale Gemisch bzw. das weitere dentale Mehrkomponentensystem zu einem Produkt härtbar ist, wobei der durch Härtung (Polymerisationshärtung) maximal erreichbare Druckmodul größer ist als 420 MPa.

Ein ganz besonders bevorzugtes erfindungsgemäßes Kit umfasst

- ein erstes dentales Gemisch oder ein erstes dentales Mehrkomponentensystem wie vorstehend definiert, wobei das erste dentale Gemisch bzw. das erste dentale Mehrkomponentensystem zu einem Produkt härtbar ist, wobei der durch Härtung maximal erreichbare Druckmodul im Bereich von 25 MPa bis kleiner 100 MPa liegt,

- ein zweites dentales Gemisch oder ein zweites dentales Mehrkomponentensystem wie vorstehend definiert, wobei das zweite dentale Gemisch bzw. das zweite dentale Mehrkomponentensystem zu einem Produkt härtbar ist, wobei der durch Härtung maximal erreichbare Druckmodul im Bereich von 100 MPa bis 420 MPa liegt,

weiter umfassend

- ein drittes dentales Gemisch oder ein drittes dentales Mehrkomponentensystem vorzugsweise wie vorstehend definiert, wobei das dritte dentale Gemisch bzw. das dritte dentale Mehrkomponentensystem zu einem Produkt härtbar ist, wobei der durch Härtung maximal erreichbare Druckmodul kleiner ist als 25 MPa, und/oder

- ein weiteres dentales Gemisch oder ein weiteres dentales Mehrkomponentensystem, vorzugsweise bestehend aus ein, zwei, oder mehr polymerisierbaren (Meth)Acrylaten sowie optional weiteren Bestandteilen, wobei das weitere dentale Gemisch bzw. das weitere dentale Mehrkomponentensystem zu einem Produkt härtbar ist, wobei der durch Härtung maximal erreichbare Druckmodul größer ist als 420 MPa.

Die Erfindung betrifft auch ein Verfahren zur Herstellung einer erfindungsgemäßen provisorischen Suprakonstruktion für ein dentales Implantat oder eines erfindungsgemäßen Kerns für eine provisorische Suprakonstruktion für ein dentales Implantat wie vorstehend erläutert oder eines erfindungsgemäßen provisorischen Zahnersatzes wie vorstehend erläutert, mit folgenden Schritten:

- Bereitstellen oder Herstellen eines erfindungsgemäß anzuwendenden dentalen Gemisches oder eines erfindungsgemäß anzuwendenden dentalen Mehrkomponentensystems,
- Härten des dentalen Gemisches oder des dentalen Mehrkomponentensystems, vorzugsweise in einer Form,
- optional weitere Schritte

oder

- Bereitstellen eines erfindungsgemäßen Kits wie vorstehend erläutert umfassend Rohlinge,
- Anpassen der Form eines der Rohlinge,

so dass die erfindungsgemäße provisorische Suprakonstruktion, der erfindungsgemäße Kern oder der erfindungsgemäße provisorische Zahnersatz resultiert.

Ein bevorzugtes erfindungsgemäßes Verfahren zur Herstellung einer provisorischen Suprakonstruktion bzw. eines entsprechenden Kerns umfasst die folgenden Schritte:

- Bereitstellen oder Herstellen eines erfindungsgemäß anzuwendenden dentalen Gemisches oder eines erfindungsgemäß anzuwendenden dentalen Mehrkomponentensystems wie vorstehend erläutert,
- Bereitstellen einer geeigneten Form,
- Befüllen der Form mit dem dentalen Gemisch oder dem dentalen Mehrkomponentensystem, und danach
- Härten (Polymerisationshärten) des dentalen Gemisches oder des dentalen Mehrkomponentensystems in der Form
- Entnahme des gehärteten Produkts (d.h. des gebildeten Rohlings, der gebildeten Suprakonstruktion oder des gebildeten Kerns) aus der Form und danach
- optional anpassen der Form des gehärteten Produkts (d.h. des so gebildeten Rohlings), sowie
- optional weitere Schritte

oder

- Bereitstellen eines erfindungsgemäßen Kits umfassend Rohlinge,
- Anpassen der Form eines der Rohlinge (z. B. durch Abschleifen eines Rohlings mit Übergröße),

so dass eine erfindungsgemäße Suprakonstruktion oder ein erfindungsgemäßer Kern resultiert.
Es versteht sich, dass vorstehend gemachte Ausführungen betreffend bevorzugte Ausgestaltungen der erfindungsgemäß anzuwendenden dentalen Gemische und dentalen Mehrkomponentensysteme, der erfindungsgemäßen provisorischen Suprakonstruktion, des erfindungsgemäßen Kerns und des erfindungsgemäßen Kits entsprechend auch für die erfindungsgemäßen Verfahren gelten.
Ein besonders bevorzugtes Verfahren zur Herstellung einer provisorischen Suprastruktur für ein dentales Implantat oder eines Kerns für eine provisorische Suprakonstruktion für ein dentales Implantat oder eines erfindungsgemäßen Zahnersatzes umfasst folgende Schritte:

- Herstellen eines Modells der dentalen Situation aus einer Modellmasse, wobei in dem Modell der bzw. die durch die provisorische dentale Suprastruktur zu ersetzenden Zähne ausgespart sind,

- Anordnen jeweils eines Manipulations-Implantats an der bzw. den Positionen des Modells, die der bzw. den Positionen des bzw. der dentalen Implantate in der dentalen Situation entsprechen,

- Anordnen je eines (als Modell dienenden oder unmittelbar in der Mundhöhle verwendbaren) Abutments auf dem bzw. jedem der besagten Manipulations-Implantate, wobei das bzw. jedes Abutment identisch oder in seinen Abmessungen identisch ist mit dem bzw. den jeweiligen, vorzugsweise vorgefertigten, handelsüblichen Abutments des eingesetzten oder einzusetzenden dentalen Implantates,

- Aufbringen einer isolierenden Schicht auf dem bzw. den Abutments, die auf dem bzw. den Manipulations-Implantaten angeordnet sind,

- Herstellen oder Anordnen einer abnehmbaren dentalen Modellation des bzw. der durch die provisorische dentale Suprastruktur zu ersetzenden Zähne auf dem bzw. den mit einer isolierenden Schicht versehenen Abutments,

- Herstellen einer Form durch Abformen des Modells inklusive der abnehmbaren dentalen Modellation,

- Abnehmen der dentalen Modellation von dem bzw. den mit einer isolierenden Schicht versehenen, auf dem bzw. den Manipulations-Implantaten angeordneten Abutments,

- erneutes Aufbringen einer isolierenden Schicht auf dem bzw. den auf dem bzw. den Manipulations-Implantaten angeordneten Abutments,

- Bereitstellen oder Herstellen eines erfindungsgemäßen anzuwendenden dentalen Gemisches oder eines erfindungsgemäßen anzuwendenden dentalen Mehrkomponentensystems wie vorstehend definiert,

- Befüllen der durch Abformen hergestellten Form mit dem bereitgestellten oder hergestellten dentalen Gemisch bzw. dem bereitgestellten oder hergestellten dentalen Mehrkomponentensystem,

- Aufsetzen der befüllten Form auf das bzw. die mit einer isolierenden Schicht versehenen, auf dem bzw. den Manipulations-Implantaten angeordneten Abutments auf dem Modell und danach Härtenlassen des dentalen Gemisches bzw. des dentalen Mehrkomponentensystems,

- vorzugsweise Abnehmen der Form mit dem gehärteten, nun eingeformten Produkt (resultierend aus dem dentalen Gemisch bzw. dem dentalen Mehrkomponentensystem durch Polymerisationshärtung) vom Modell,

- Trennen der aus dem gehärteten eingeformten Produkt gebildeten provisorischen dentalen Suprastruktur oder des gebildeten Kerns von der Form.

Es versteht sich, dass in den erfindungsgemäßen Verfahren dentale Implantate vorzugsweise in Kombination mit handelsüblichen, vom Implantathersteller für das entsprechende Implantat vorgefertigten Abutments eingesetzt werden können. Zu bevorzugten Ausgestaltungen von Abutments verweisen wir auf unsere vorstehenden Erläuterungen, die auch für das erfindungsgemäße Verfahren und dessen bevorzugte Ausgestaltungen zutreffen.

Der Begriff "dentale Situation" bezeichnet die Situation in der Mundhöhle eines Patienten, insbesondere eines Patienten, welcher mit einer dentalen Restauration auf Basis eines Implantats versorgt werden soll.

Der Begriff "Manipulations-Implantate" bezeichnet formgetreue Nachbildungen des an der entsprechenden Position in der Mundhöhle eingesetzten oder einzusetzenden dentalen Implantats.

Die in vorstehend beschriebenen bevorzugten erfindungsgemäßen Verfahren verwendete isolierende Schicht auf dem bzw. den auf dem bzw. den Manipulationsimplantaten angeordneten Abutments bewirkt insbesondere, dass auf dem als Modell dienenden bzw. unmittelbar in der Mundhöhle verwendbaren Abutment eine passgenaue aber nicht anhaftende Suprakonstruktion hergestellt werden kann. Die Suprakonstruktion darf in dem entsprechenden Prozessstadium noch nicht fest an dem Abutment anhaften, denn bei einem festen Verbund aus Abutment und Suprakonstruktion wäre es natürlich nicht mehr möglich, das Abutment in das in der Mundhöhle des Patienten eingesetzte Implantat in üblicher Weise einzuschrauben.

Das erfindungsgemäße Verfahren zur Herstellung einer provisorischen Suprakonstruktion, eines Kerns zu einer provisorischen Suprakonstruktion bzw. eines provisorischen Zahnersatzes kann bevorzugt als sogenanntes chairside-Verfahren ausgestaltet werden. Ein erfindungsgemäßes (chair-side-)Verfahren zur Herstellung einer provisorischen Suprakonstruktion für ein dentales Implantat oder eines Kerns zu einer provisorische Suprakonstruktion für ein dentales Implantat wie vorstehend definiert oder eines provisorischen Zahnersatzes wie vorstehend definiert, wobei für das bzw.

jedes dentale Implantat ein Abutment zur Verbindung der provisorischen Suprakonstruktion mit dem dentalen Implantat vorgesehen ist, umfasst die folgenden Schritte:

- Herstellen eines Modells der dentalen Situation, in der sich der bzw. die durch die provisorische dentale Suprakonstruktion zu ersetzenden Zähne noch im Mund des Patienten befinden, aus einer Modellmasse, so dass das Modell den bzw. die durch die provisorische dentale Suprakonstruktion zu ersetzenden Zähne nachbildet,

- Einsetzen des bzw. der dentalen Implantate mit dem jeweils dazugehörigen Abutment im Mund des Patienten,

- optional Aufbringen einer isolierenden Schicht auf einem bzw. mehreren (Modell-)Abutments des bzw. der eingesetzten dentalen Implantate im Mund des Patienten,

- Herstellen einer Form durch Abformen des Modells,

- Bereitstellen oder Herstellen eines erfindungsgemäß anzuwendenden dentalen Gemisches oder eines erfindungsgemäß anzuwendenden dentalen Mehrkomponentensystems,

- Befüllen der durch Abformen hergestellten Form mit dem dentalen Gemisch oder dem dentalen Mehrkomponentensystem,

- Aufsetzen der befüllten Form auf das bzw. die gegebenenfalls mit einer isolierenden Schicht versehenen (Modell-)Abutments des bzw. der im Mund des Patienten eingesetzten dentalen Implantate und danach Härtenlassen des dentalen Gemisches oder des dentalen Mehrkomponentensystems (welches als Ausgangsmaterial zur Herstellung der provisorischen Suprakonstruktion oder des Kerns dient),

- in manchen Fällen insbesondere zum Zwecke der Nachbearbeitung Abnehmen der Form mit dem gehärteten, nun eingeformten Produkt von dem bzw. den mit einer isolierenden Schicht versehenen (Modell-)Abutments,

- Trennen der aus dem gehärteten eingeformten Produkt gebildeten provisorischen Suprakonstruktion von der Form.

Es versteht sich, dass das erfindungsgemäße Verfahren zwar als chair-side-Verfahren ausgestaltet sein kann, jedoch nicht als chair-side-Vefahren ausgebildet sein muss. In einer Vielzahl von Fällen ist es vorteilhaft, anstelle eines chair-side-Verfahrens eine Verfahrensgestaltung zu wählen, bei der kein einzelner Verfahrensschritt im Mund des oder am Patienten erfolgt. Derartige Verfahrensgestaltungen werden auch als lab-side-Verfahren bezeichnet.

Sofern in erfindungsgemäßen Verfahren zur Herstellung einer provisorischen Suprakonstruktion bzw. eines Kerns für eine provisorische Suprakonstruktion vorgefertigte Abutments eingesetzt werden, ist es vorteilhaft, wenn das erfindungsgemäße Verfahren den folgenden weiteren Schritt umfasst:

- Bereitstellen eines vorgefertigten Abutments für das einzusetzende oder eingesetzte dentale Implantat bzw. je eines vorgefertigten Abutments für jedes einzusetzende oder eingesetzte dentale Implantat sowie gegebenenfalls eines zweiten vorgefertigten Abutments für das einzusetzende oder eingesetzte dentale Implantat bzw. je eines zweiten vorgefertigten Abutments für jedes einzusetzende oder eingesetzte dentale Implantat, wobei das bzw. das jeweilige zweite Abutment in seinen Abmessungen identisch mit dem bzw. dem jeweiligen vorgefertigten Abutment des oder des jeweiligen eingesetzten oder einzusetzenden dentalen Implantats ist.

Die in bevorzugten erfindungsgemäßen Verfahren auf (Modell-)Abutments anzubringende isolierende Schicht umfasst vorzugsweise Vaseline oder Glyzerin oder besteht aus diesen Substanzen.

Die in bevorzugten erfindungsgemäßen Verfahren einzusetzende abnehmbare dentale Modellation wird vorzugsweise in Form eines Wax-Up-Modells bzw. aus lichthärtendem Kunststoff hergestellt.

Die in bevorzugten erfindungsgemäßen Verfahren einzusetzende Form ist gemäß einer ersten Alternative

(a) eine durch Tiefziehen einer Tiefziehfolie über das Modell hergestellte Modellschiene oder wird gemäß einer zweiten Alternative

(b) durch Abformen mittels eines abformfähigen Kunststoffs (z.B. aus Silikonmaterial) von dem Modell hergestellt.

Die Form ist vorzugsweise transparent, so dass eine Bestrahlung von in die Form eingefülltem dentalen Gemisch oder dentalen Mehrkomponentensystem mit Licht durch das Formmaterial hindurch möglich ist. Vorzugsweise ist ein erfindungsgemäß einzusetzendes dentales Gemisch oder Mehrkomponentensystem licht- oder dualhärtend und wird in der Form mittels Bestrahlung durch das Formmaterial hindurch gehärtet (Polymerisationshärtung).

Es versteht sich, dass die in bevorzugten erfindungsgemäßen Verfahren hergestellten gehärteten Produkte zur Erzeugung einer für den unmittelbaren Einsatz geeigneten dentalen Suprakonstruktion bzw. eines entsprechenden Kerns noch nachbearbeitet werden können. Erfindungsgemäße Verfahren umfassen deshalb in manchen Fällen als weiterer Schritt das Nachbearbeiten der gehärteten, eingeformten Produkte bzw. der gebildeten provisorischen Suprakonstruktion bzw. des Kerns, wobei das Nachbearbeiten bevorzugt mittels einer oder mehrerer Techniken ausgewählt aus der Gruppe bestehend aus Trimmen, Fräsen und Polieren erfolgt.

Aus den Erläuterungen weiter oben ergibt sich, dass in den erfindungsgemäßen Verfahren vorzugsweise das bzw. die eingesetzten Abutments keine Unter- oder Hinterschneidungen besitzen.

Erfindungsgemäße provisorische Suprakonstruktionen für ein dentales Implantat sind vorzugsweise ausgewählt aus der Gruppe bestehend aus Kronen und Brücken. Entsprechend sind erfindungsgemäße Kerne vorzugsweise Kerne für eine provisorische Krone oder Brücke.

In bevorzugten erfindungsgemäßen Verfahren zur Herstellung einer provisorischen Suprakonstruktion bzw. eines entsprechenden Kerns werden mehrere individuelle Formen hergestellt. Jede dieser individuellen Formen dient dann dazu, einzelne der nacheinander zu verwendenden provisorischen dentalen Suprakonstruktionen herzustellen, die eine ansteigende Härte (ansteigende Druckmoduln, siehe oben) besitzen sollen.

In bevorzugten erfindungsgemäßen Verfahren wird wie erwähnt eine Form für die Herstellung der erfindungsgemäßen provisorischen dentalen Suprakonstruktion eingesetzt. Ein (Teil-)Verfahren zur Herstellung dieser Form umfasst in einer bevorzugten Ausgestaltung die folgenden Schritte:

- Herstellen eines Modells einer dentalen Situation aus einer Modellmasse, wobei im Modell der bzw. die durch die provisorische Suprakonstruktion zu ersetzenden Zähne ausgespart sind,

- Anordnen jeweils eines Manipulations-Implantats an der bzw. den Positionen des Modells, die der bzw. den Positionen des bzw. der dentalen Implantate in der dentalen Situation entsprechen,

- Anordnen je eines (Modell-)Abutments auf dem bzw. jedem der besagten Manipulations-Implantate, wobei das bzw. jedes Abutment identisch oder in seinen Abmessungen identisch mit dem bzw. den jeweiligen vorgefertigten Abutment des eingesetzten oder einzusetzenden dentalen Implantates ist,

- Aufbringen einer isolierenden Schicht auf dem bzw. den auf dem bzw. den Manipulations-Implantaten angeordneten (Modell-)Abutments,

- Herstellen oder Anordnen einer abnehmbaren dentalen Modellation des bzw. der durch die provisorische Suprakonstruktion zu ersetzenden Zähne auf dem bzw. den mit einer isolierenden Schicht versehenen (Modell-)Abutments,

- Herstellen einer Form durch Abformen des Modells inklusive der abnehmbaren dentalen Modellation.

Beschrieben wird auch ein Verfahren zur provisorischen Versorgung eines dentalen Implantats für therapeutische oder kosmetische Zwecke, umfassend die Schritte

(a) Bereitstellen einer erfindungsgemäßen provisorischen Suprakonstruktion wie vorstehend definiert oder Bereitstellen mehrerer erfindungsgemäßer provisorischer Suprakonstruktion wie vorstehend definiert und Auswählen einer provisorischen Suprakonstruktion,
oder
Bereitstellen mehrerer provisorischer Suprakonstruktionen wie vorstehend definiert und Auswählen einer provisorischen Suprakonstruktion,

(b) Verbinden der provisorischen Suprakonstruktion mit dem dentalen Implantat (vorzugsweise mittels eines Abutments) und danach

(c) Verstreichenlassen einer gewissen Zeitspanne und danach

(d) Lösen der (vorzugsweise mittels eines Abutments hergestellten) Verbindung zwischen der provisorischen Suprakonstruktion und dem dentalen Implantat (wobei das vorzugsweise eingesetzte Abutment vorteilhafterweise mit dem Implantat verbunden bleibt)

und gegebenenfalls danach die folgenden Schritte:

(e) Auswählen einer weiteren in Schritt (a) bereitgestellten provisorischen Suprakonstruktion,

(f) Verbinden der in Schritt (e) ausgewählten provisorischen Suprakonstruktion mit dem dentalen Implantat und danach

(g) Verstreichenlassen einer gewissen Zeitspanne und danach

(h) Lösen der (vorzugsweise mittels eines Abutments hergestellten) Verbindung zwischen der provisorischen Suprakonstruktion und dem dentalen Implantat (wobei das vorzugsweise eingesetzte Abutment vorteilhafterweise mit dem Implantat verbunden bleibt),

(i) Wiederholen der Schritte (e) bis (h), bis mehrere oder sämtliche der in Schritt (a) bereitgestellten provisorischen Suprakonstruktionen mit dem Implantat verbunden worden sind und die jeweilige Verbindung wieder gelöst worden ist.

Das Verfahren zur provisorischen Versorgung eines dentalen Implantats für kosmetische Zwecke wird vorzugsweise mit nur einer erfindungsgemäßen provisorischen Suprakonstruktion wie vorstehend definiert durchgeführt.

Bei einem bevorzugten Verfahren zur provisorischen therapeutischen Versorgung eines dentalen Implantats werden mehrere provisorische Suprakonstruktionen bestehend aus bzw. aus Kernmaterial umfassend Materialien mit unterschiedlichen Druckmoduln bereitgestellt oder hergestellt und in der Reihenfolge ansteigender Druckmoduln zeitlich nacheinander mit dem dentalen Implantat verbunden.

In einem besonderes bevorzugten Verfahren zur provisorischen Versorgung eines dentalen Implantats für therapeutische und/oder kosmetische Zwecke werden die, mehrere oder sämtliche der provisorischen Suprakonstruktionen so mit dem dentalen Implantat verbunden, dass die jeweilige provisorische Suprakonstruktion bei Kieferschluss in Okklusion mit der Antagonistenbezahnung steht.

Beschrieben wird schließlich auch die Verwendung eines erfindungsgemäß anzuwendenden dentalen Gemisches oder eines erfindungsgemäß anzuwendenden dentalen Mehrkomponentensystems wie vorstehend definiert zum Herstellen einer provisorischen Suprakonstruktion für ein dentales Implantat oder eines Kerns für eine provisorische Suprakonstruktion für ein dentales Implantat, vorzugsweise einer erfindungsgemäßen provisorischen Suprakonstruktion oder eines erfindungsgemäßen Kerns wie vorstehend definiert.

Weitere Aspekte der vorliegenden Erfindung ergeben sich aus den nachfolgenden Vorschriften, Beispielen und Patentansprüchen.

Vorschrift zur Bestimmung des durch Härtung maximal erreichbaren Druckmoduls eines dentalen Gemisches oder dentalen Mehrkomponentensystems:

1. Herstellen eines Probekörpers:

Das dentale Gemisch bzw. das dentale Mehrkomponentensystem dessen durch Härtung maximal erreichbarer Druckmodul zu bestimmen ist, wird bereitgestellt. Im Falle eines Mehrkomponentensystems werden die Komponenten vollständig durchmischt, so dass sich ein dentales Gemisch bildet.

Es wird nun gehärtet, wobei die Härtungsbedingungen von den Eigenschaften der zu untersuchenden Substanz abhängen:

- Sofern das bereitgestellte oder hergestellte dentale Gemisch durch Bestrahlung mit Licht bis zum maximal erreichbaren Druckmodul härtbar ist, wird es so mit Licht bestrahlt, dass sein maximal erreichbarer Druckmodul erreicht wird.

- Sofern das bereitgestellte oder hergestellte dentale Gemisch nicht durch Bestrahlung mit Licht, jedoch durch chemische Härtung bis zum maximal erreichbaren Druckmodul härtbar ist, wird erforderlichenfalls nach Zusatz von Initiatoren eine chemische Härtung initiiert, so dass der maximal erreichbare Druckmodul erreicht wird.

- Ist das bereitgestellte dentale Gemisch nicht durch Bestrahlung mit Licht und auch nicht durch chemische Härtung bis zum maximal erreichbaren Druckmodul härtbar, sondern lediglich durch eine Kombination einer Bestrahlung mit Licht und einer chemischen Härtung, so wird das bereitgestellte dentale Gemisch sowohl mit Licht bestrahlt als auch eine chemische Härtung initiiert, so dass der maximal erreichbare Druckmodul erreicht wird.

Es ist hierbei anhand von Vorversuchen zu ermitteln, welche Härtungsbedingungen zu wählen sind, um den maximal erreichbaren Druckmodul sicher zu erreichen.

Das Härten des bereitgestellten dentalen Gemisches zu einem zylinderförmigen Probekörper wird in einer Teflonform mit zylindrischem Durchgangsloch und einem Innendurchmesser von 4,0 mm sowie einer Höhe von 6,0 mm durchgeführt. Das bereitgestellte dentale Gemisch wird in die Form gefüllt und auf beiden freien Seiten mit einer lichtdurchlässigen Acetatfolie bedeckt. Anschließend wird das bereitgestellte dentale Gemisch in der Form unter den oben angegebenen Härtungsbedingungen gehärtet, so dass ein zylinderförmiger Probekörper mit einem Durchmesser von 4 mm und einer Höhe von 6 mm resultiert. Die exakten Dimensionen (Höhe = $L_0$ und Durchmesser = $d_0$) des Probekörpers werden mit einer Messschraube oder einem entsprechenden Messinstrument mit einer Skalenteilung von 2 $\mu$m oder weniger bestimmt.

2. Bestimmung des Druckmoduls des hergestellten Probekörpers:

Zur Bestimmung des Druckmoduls wird zunächst die Druckfestigkeit bestimmt. Aus den ermittelten Messdaten wird der Druckmodul berechnet.

Die Bestimmung der Druckfestigkeit erfolgt in Anlehnung an EN ISO 9917 Teil 1.

Der gemäß Punkt 1 hergestellte zylinderförmige Probekörper wird zur Vorbereitung der Messung mit den ebenen Flächen zwischen die Druckstempel einer Universalprüfmaschine gebracht und bei einer Vorschubgeschwindigkeit von 3 mm/min bis zu einer Vorkraft von 5 Newton belastet. Der bis zum Erreichen der besagten Vorkraft zurückgelegte Vorschubweg wird nachfolgend nicht berücksichtigt.

Zur Bestimmung der Druckfestigkeit (siehe nachfolgend (a)) und zur Bestimmung des Druckmoduls (siehe nachfolgend (b)) wird der Prüfkörper anschließend bei einer Prüfgeschwindigkeit von 0,5 mm/min bis zum Bruch belastet; dabei wird die Kraft (F) als Funktion des Vorschubweges (L), in mm, aufgezeichnet (ausgehend von L = 0 mm bei F = 5 N, siehe oben). Insgesamt werden mindestens fünf Probekörper hergestellt, an denen jeweils eine Messung durchgeführt wird:

(a) Die beim Bruch des jeweiligen Probekörpers ausgeübte Höchstkraft ($F_{max}$) wird notiert und die <u>Druckfestigkeit</u> ($\sigma$) wird in Megapascal (MPa) anhand der folgenden Gleichung berechnet:

$$\sigma = \frac{4 * F_{max}}{\pi * d_0^2}$$

Dabei ist

$F_{max}$: die beim Bruch gemessene Höchstkraft, in Newton (N);
$d_0$: der gemessene Durchmesser des Probekörpers, in mm.

(b) Die bei der Messung der Druckfestigkeit ermittelte Kraft (F) in Newton, wird als Funktion des Vorschubweges (L) in mm aufgetragen. Im Bereich eines Vorschubweges zwischen 0,2 mm und 0,5 mm wird der lineare Kurvenbereich bestimmt. Anschließend werden die Anfangskoordinaten ($L_A$ und $F_A$) und die Endkoordinaten ($F_E$ und $L_E$) des linearen Kurvenbereiches ermittelt. Mit den so ermittelten Werten wird der <u>Druckmodul</u> (E), in Megapascal (MPa), anhand der folgenden Gleichung berechnet:

$$E = \frac{[4 * L_0 * (F_E - F_A)]}{[\pi * d_0^2 * (L_E - L_A)]}$$

Dabei ist

$L_0$: die gemessene Höhe des Probekörpers, in mm;
$d_0$: der gemessene Durchmesser des Probekörpers, in mm;
$F_A$: Kraft am Anfang des linearen Bereichs, in Newton (N);
$F_E$: Kraft am Ende des linearen Bereichs, in Newton (N);
$L_A$: Vorschubweg am Anfang des linearen Bereichs, in mm;
$L_E$: Vorschubweg am Ende des linearen Bereichs, in mm.

Aus den zumindest fünf durchgeführten Bestimmungen des Druckmoduls wird der Mittelwert gebildet. Dieser Mittelwert für den Druckmodul ist der Wert für den durch Härtung maximal erreichbaren Druckmodul.

Im Rahmen des vorliegenden Textes wird davon ausgegangen, dass der Druckmodul einer provisorischen Suprakonstruktion, eines Kerns für eine provisorische Suprakonstruktion bzw. eines entsprechenden Rohlings dem Druckmodul des Materials entspricht, aus dem es hergestellt wurde.

Es sei darauf hingewiesen, dass sich der Druckmodul einer provisorischen Suprakonstruktion, eins Kerns für eine provisorische Suprakonstruktion bzw. des Materials eines entsprechenden Rohlings in der Regel in der Mundhöhle des Patienten ändern wird. In der Regel ist der Druckmodul der resultierenden "nassen" Produkte kleiner als der der "trockenen" Produkte.

**Beispiele:**

Anhand der folgenden Beispiele wird die Erfindung weiter erläutert. Sofern nicht anders angegeben, beziehen sich alle Angaben auf das Gewicht. Es werden dabei folgende Abkürzungen verwendet:

UDMA = Urethandimethacrylat (CAS Number 72869-86-4)

aliphatisches Urethandimethacrylat = Genomer 4256 von Rahn AG

Polyethylenglykoldimethacrylat = Polyethylenglykol 400-dimethacrylat (n= 9)

Coinitiator = Ethyl-*p-N,N*-dimethylaminobenzoat

Stabilisator = 2,6-Di-*tert*-butyl-4-methylphenol

Präpolymerisat = polymerisierte Alkyldimethacrylate

GK = silanisiertes Bariumaluminimsilikatglas ($d_{50}$=1,0 $\mu$m)

UV-Stabilisator = 2-(2H-benzotriazol-2-yl)-*p*-cresol

Pyrogene Kieselsäure = pyrogene Kieselsäure mit einer BET-Oberfläche im Bereich von: 170-230 $m^2$/g

Beispiele A bis F: Herstellen erfindungsgemäßer dentaler Gemische und Mehrkomponentensysteme

Beispiel A: Herstellen eines erfindungsgemäßen dentalen Gemisches

Die folgenden Komponenten wurden in ein Becherglas eingewogen:

| | | |
|---|---|---|
| aliphatisches Urethandimethacrylat | 50,00 g | Komponente a) |
| Polyethylenglykoldimethacrylat | 49,65 g | Komponente a) |
| Campherchinon | 0,10 g | Komponente b) |
| Coinitiator | 0,15 g | Komponente b) |
| Stabilisator | 0,10 g | Komponente d) |

Anschließend wurde das Gemisch mit einem KPG Rührer für 12 Stunden homogenisiert und anschließend für zwei Stunden im Exsiccator entlüftet.

Beispiel B: Herstellen eines erfindungsgemäßen dentalen Gemisches

Die folgenden Komponenten wurden in ein Becherglas eingewogen:

| | | |
|---|---|---|
| aliphatisches Urethandimethacrylat | 79,83 g | Komponente a) |
| UDMA | 8,65 g | Komponente a) |
| Hexandioldimethacrylat | 11,20 g | Komponente a) |
| Campherchinon | 0,12 g | Komponente b) |
| Coinitiator | 0,19 g | Komponente b) |

(fortgesetzt)

| Stabilisator | 0,10 g | Komponente d) |
|---|---|---|

Anschließend wurde das Gemisch mit einem KPG Rührer für 12 Stunden homogenisiert und anschließend für zwei Stunden im Exsiccator entlüftet.

Beispiel C: Herstellen eines erfindungsgemäßen dentalen Gemisches in Pasteform:

Die folgenden Komponenten wurden in ein Becherglas eingewogen:

| aliphatisches Urethandimethacrylat | 45,56 g | Komponente a) |
|---|---|---|
| UDMA | 4,92 g | Komponente a) |
| Hexandioldimethacrylat | 6,36 g | Komponente a) |
| Campherchinon | 0,07 g | Komponente b) |
| Coinitiator | 0,1 g | Komponente b) |
| Stabilisator | 0,06 g | Komponente d) |
| Pigmente | 0,04 g | Komponente e) |

Anschließend wurde das Gemisch mit einem KPG Rührer für 12 Stunden homogenisiert und für zwei weitere Stunden im Exsiccator entlüftet. Zu dieser Mischung wurden die Füllstoffe:

| Präpolymerisat | 27,06 g | Komponente c) |
|---|---|---|
| Pyrogene Kieselsäure | 15,83 g | Komponente c) |

hinzugegeben und durch inniges Vermischen mit einem Doppelplaneten-Mischer, wurde eine homogene Paste hergestellt.

Beispiel D: Herstellen eines erfindungsgemäßen dentalen Gemisches

Die folgenden Komponenten wurden in ein Becherglas eingewogen:

| aliphatisches Urethandimethacrylat | 30,34 g | Komponente a) |
|---|---|---|
| UDMA | 7,45 g | Komponente a) |
| Hexandioldimethacrylat | 3,76 g | Komponente a) |
| Bis-GMA | 3,69 g | Komponente a) |
| Triethylenglykoldimethacrylat | 1,27 g | Komponente a) |
| Campherchinon | 0,07 g | Komponente b) |
| Coinitiator | 0,10 g | Komponente b) |
| Stabilisator | 0,05 g | Komponente d) |
| GK | 22,81 g | Komponente c) |
| UV-Stabilisator | 0,04 g | Komponente d) |
| Pigmente | 0,03 g | Komponente e) |

Anschließend wurde das Gemisch mit einem KPG Rührer für 12 Stunden homogenisiert und für zwei weitere Stunden im Exsiccator entlüftet. Zu dieser Mischung wurden die Füllstoffe:

| Pyrogene Kieselsäure | 12,36 g | Komponente c) |
|---|---|---|
| Präpolymerisate | 18,04 g | Komponente c) |

hinzugegeben und durch inniges Vermischen mit einem Doppelplaneten-Mischer, wurde eine homogene Paste hergestellt.

Beispiel E: Herstellen eines erfindungsgemäßen dentalen Gemisches

Für die Herstellung der erfindungsgemäßen dentalen Gemische wurden die folgenden Komponenten in ein Becherglas eingewogen:

| | | |
|---|---|---|
| aliphatisches Urethandimethacrylat | 75,45 g | Komponente a) |
| Polyethylenglykoldimethacrylat | 24,20 g | Komponente a) |
| Campherchinon | 0,10 g | Komponente a) |
| Coinitiator | 0,15 g | Komponente d) |
| Stabilisator | 0,11 g | Komponente b) |

Anschließend wurde das Gemisch mit einem KPG Rührer für 12 Stunden homogenisiert und für zwei weitere Stunden im Exsiccator entlüftet.

Beispiel F: Herstellung eines erfindungsgemäßen dentalen Mehrkomponentensystems

Beispiel F1: Herstellung des Katalysator-Gemisches

Für die Herstellung des Katalysator-Gemisches wurden die folgenden Komponenten in ein Becherglas eingewogen:

| | | |
|---|---|---|
| aliphatisches Urethandimethacrylat | 48,78 g | Komponente a) |
| Polyethylenglykoldimethacrylat | 48,96 g | Komponente a) |
| Stabilisator | 0,12 g | Komponente b) |
| Benzoylperoxid | 2,14 g | Komponente b) |

Anschließend wurde das Gemisch mit einem KPG Rührer für 12 Stunden homogenisiert und für weitere 2 Stunden im Exsiccator entlüftet.

Beispiel F2: Herstellung des Basis-Gemisches

Für die Herstellung des Basis-Gemisches wurden die folgenden Komponenten in ein Becherglas eingewogen:

| | | |
|---|---|---|
| aliphatisches Urethandimethacrylat | 48,84 g | Komponente a) |
| Polyethylenglykoldimethacrylat | 48,84 g | Komponente a) |
| *N,N*-Bis(2-hydroxyethyl)-*p*-toluidin | 1,53 g | Komponente b) |
| Campherchinon | 0,31 g | Komponente b) |
| Coinitiator | 0,45 g | Komponente b) |

Das Gemisch wurde mit einem KPG Rührer für 12 Stunden homogenisiert und für weitere 2 Stunden im Exsiccator entlüftet.

Beispiel G: Bestimmung von Materialeigenschaften der in den Beispielen A bis F hergestellten dentalen Gemische und dentalen Mehrkomponentensysteme:

Die in den Beispielen A bis E hergestellten erfindungsgemäßen dentalen Gemische bzw. das dentale Mehrkomponentensystem gemäß Beispiel F (nach Mischung der Komponenten im Volumenverhältnis 1:1) wurde entsprechend der obigen "Vorschrift zur Bestimmung des durch Härtung maximal erreichbaren Druckmoduls eines dentalen Gemisches oder dentalen Mehrkomponentensystems" auf ihren Druckmodul untersucht. Teilmengen des dentalen Mehrkomponentensystems gemäß Beispiel F wurden zu Testzwecken sowohl mit Lichteinstrahlung (dual-cure), als auch ohne Lichteinstrahlung (chemische Härtung) gehärtet. Die Probekörper wurden nach dem Entformen bei 37°C trocken gelagert und anschließend vermessen. Zum Vergleich wurde das selbsthärtende provisorische Kronenmaterial Structur 2 SC der VOCO GmbH untersucht. Die Messwerte sind in der folgenden Tabelle zusammengefasst:

| Beispiel | Druckfestigkeit [MPa] | Druckmodul [MPa] | Dehnbarkeit [mm] | max. Kraft [N] | Kraft bei 1 mm [N] |
|---|---|---|---|---|---|
| A trocken | 58 | 21 | 3,0 | 308 | 53 |
| B trocken | 112 | 16 | 2,4 | 60 | 53 |
| C trocken | 45 | 92 | 3,0 | 370 | 108 |
| D trocken | 96 | 400 | 1,6 | 920 | 619 |
| E trocken | 44 | 4 | 3,0 | 105 | 29 |
| F trocken DC* | 226 | 11 | 2,82 | 323 | 60 |
| trocken CC# | 221 | 14 | 2,75 | 253 | 59 |
| *Structur 2 SC* | 277 | 1170 | n.b.♣ | n.b. | n.b. |
| *DC: dual gehärtet (Licht+chemisch); #CC: chemisch gehärtet; ♣ Die Dehnbarkeit konnte aufgrund der Härte des Materials nicht bestimmt werden; n.b.: nicht bestimmt | | | | | |

Die Ergebnisse der Untersuchungen zeigen, dass die erfindungsgemäßen Beispiele A-F zu Produkten/Materialeien geführt haben, die hervorragend in der progressive bone loading Therapie als Material von Suprakonstruktionen eingesetzt werden können.

Beispiel H: Untersuchung zur Haftung gehärteter erfindungsgemäßer dentaler Gemische und dentaler Mehrkomponentensysteme an metallischen Substraten (Zugversuch):

Um die Haftung der erfindungsgemäßen dentalen Suprakonstruktionen, an metallischen Abutments, mit der Haftung von Suprakonstruktion aus bekannten Materialien an metallischen Abutments zu vergleichen, wurden Suprakonstruktionstestkörper auf einem Abutmenttestkörper aus Edelstahl hergestellt.

Die Suprakonstruktionstestkörper bestanden dabei aus:

a) einem handelsüblichen Siliconmaterial (additionsvernetzendes Silikon; Fit Test (VOCO), Druckmodul < 420 MPa),

b) einem handelsüblichen Methacrylatmaterial (kaltpolymerisierendes Paste-Paste-System; Structur 2 SC (VOCO)),

c) einem erfindungsgemäßen dentalen Mehrkomponentensystem gemäß Beispiel F und

d) einem erfindungsgemäßen dentalen Gemisch gemäß Beispiel D.

Anschließend wurde jeweils die Abzugskraft mit Hilfe einer Universalprüfmaschine gemessen, indem das Modellabutment aus der Suprakonstruktion gezogen wurde. Dabei konnten die folgenden Werte für die Abzugskraft bestimmt werden:

| Probe | Abzugskraft [MPa] |
|---|---|
| a) | 0,33 |
| b) | 2,95 |
| c) | 0,68* |

(fortgesetzt)

| Probe | Abzugskraft [MPa] |
|---|---|
| d) | 2,34 |
| * Bei Probe c) wurde nicht das Metallabutment aus der Suprakonstruktion gezogen, sondern das Material der Suprakonstruktion riss. | |

**Patentansprüche**

1. Dentales Gemisch oder dentales Mehrkomponentensystem zur Herstellung des Materials einer provisorischen Suprakonstruktion für ein dentales Implantat oder eines Kerns für eine provisorische Suprakonstruktion für ein dentales Implantat durch Polymerisationshärtung, bestehend aus

   (a) insgesamt 20,0 bis 99,9 Gewichtsprozent an ein, zwei oder mehr polymerisierbaren (Meth)Acrylaten,
   (b) insgesamt 0,1 bis 10,0 Gewichtsprozent an einem oder mehreren Katalysatoren und/oder Initiatoren für die Polymerisation,
   (c) insgesamt 0,0 bis 60,0 Gewichtsprozent an einem oder mehreren anorganischen und/oder organischen Füllstoffen,
   (d) insgesamt 0,0 bis 5,0 Gewichtsprozent an einem oder mehrerem Stabilisatoren, für die gilt: der oder die Stabilisatoren sind keine polymerisierbaren (Meth)Acrylate, sowie
   (e) insgesamt 0,0 bis 50,0 Gewichtsprozent an einem oder mehreren weiteren Additiven,

   wobei das dentale Gemisch bzw. das Mehrkomponentensystem zu einem Produkt härtbar ist,
   zur Anwendung in einer therapeutischen Behandlung zur Beschleunigung der Osseointegration dentaler Implantate nach der Methode des progressive bone loading, wobei

   - aus dem dentalen Gemisch bzw. dem Mehrkomponentensystem eine provisorische Suprakonstruktion für ein dentales Implantat oder ein Kern für eine provisorische Suprakonstruktion für ein dentales Implantat durch Polymerisationshärtung hergestellt wird

   oder

   - im Rahmen der Behandlung mehrere provisorische Suprakonstruktionen bestehend aus bzw. als Kernmaterial umfassend Materialien mit unterschiedlichen Druckmoduln hergestellt und in der Reihenfolge ansteigender Druckmoduln zeitlich nacheinander mit dem dentalen Implantat verbunden werden.

2. Dentales Gemisch oder dentales Mehrkomponentensystem zur Anwendung in einer therapeutischen Behandlung zur Beschleunigung der Osseointegration dentaler Implantate nach der Methode des progressive bone loading nach Anspruch 1, umfassend in oder als Komponente (c) einen oder mehrere partikuläre anorganische und/oder organische Füllstoffe, vorzugsweise ausgewählt aus der Gruppe bestehend aus Siliciumdioxid, Metalloxiden und Poly(meth)acrylaten.

3. Provisorische Suprakonstruktion für ein dentales Implantat oder Kern für eine provisorische Suprakonstruktion für ein dentales Implantat, herstellbar durch Polymerisationshärtung eines dentalen Gemisches oder eines Mehrkomponentensystems wie in einem der Ansprüche 1 oder 2 definiert.

4. Provisorische Suprakonstruktion oder Kern für eine provisorische Suprakonstruktion nach Anspruch 3, wobei das Material der Suprakonstruktion bzw. das Material des Kerns einen Druckmodul von weniger als 420 MPa besitzt.

5. Provisorische Suprakonstruktion nach einem der Ansprüche 3 oder 4, bestehend aus einem Kern und einer Kappe für den Kern, wobei die Außenfläche des Kerns teilweise frei liegt, so dass der Kern bei Druckbelastung zumindest abschnittsweise unabhängig von der Kappe deformierbar ist, wobei das Material der Kappe vorzugsweise einen höheren Druckmodul aufweist als das Material des Kerns.

6. Provisorische Suprakonstruktion nach einem der Ansprüche 3 bis 5, herstellbar durch eine einzelne Polymerisationshärtung eines einzelnen dentalen Gemisches wie in einem der Ansprüche 1 oder 2 definiert.

7. Provisorischer Zahnersatz umfassend

   - eine provisorische Suprakonstruktion nach einem der Ansprüche 3 bis 6,
   - ein dentales Implantat

   und

   - ein vorzugsweise metallisches Abutment, das zwischen provisorischer Suprakonstruktion und dentalem Implantat angeordnet ist und deren Verbindung gewährleistet

   sowie optional

   - einen Haftvermittler, der zwischen provisorischer Suprakonstruktion und Abutment angeordnet ist.

8. Provisorischer Zahnersatz nach Anspruch 7, wobei die provisorische Suprakonstruktion und das Abutment verbunden sind ohne Formschluss in Kraftrichtung entlang der Längsachse des dentalen Implantats vom Kieferknochen weg, in den das Implantat eingesetzt oder einzusetzen ist.

9. Kit zur Herstellung einer Mehrzahl von provisorischen Suprakonstruktionen für ein dentales Implantat oder einer Mehrzahl von Kernen für provisorische Suprakonstruktionen für ein dentales Implantat, umfassend

   - eine entsprechende Anzahl dentaler Gemische oder dentaler Mehrkomponentensysteme wie in einem der Ansprüche 1 oder 2 definiert, wobei jedes der dentalen Gemische oder Mehrkomponentensysteme zu einem Produkt härtbar ist, wobei der Druckmodul der Produkte bei gleichen Härtungsbedingungen unterschiedlich ist

   oder

   - eine entsprechende Anzahl von Rohlingen aus einem gehärteten bzw. durch Härtung herstellbar aus einem dentalen Gemisch wie in einem der Ansprüche 1 oder 2 definiert, wobei der Druckmodul des Materials jedes der Rohlinge unterschiedlich ist und jeweils 420 MPa oder weniger beträgt,

   sowie

   - optional ein oder mehrere weitere Bestandteile ausgewählt aus der Gruppe bestehend aus Haftvermittler, Abutments, Implantate, Formwerkzeuge (Formen), Abformmaterialien.

10. Kit nach Anspruch 9, umfassend

   - ein erstes dentales Gemisch oder ein erstes dentales Mehrkomponentensystem wie in einem der Ansprüche 1 oder 2 definiert, wobei das erste dentale Gemisch bzw. das erste dentale Mehrkomponentensystem zu einem Produkt härtbar ist, wobei der durch Härtung (Polymerisationshärtung) ma-ximal erreichbare Druckmodul im Bereich von 25 MPa bis kleiner 100 MPa liegt,
   - ein zweites dentales Gemisch oder ein zweites dentales Mehrkomponentensystem wie in einem der Ansprüche 1 oder 2 definiert, wobei das zweite dentale Gemisch bzw. das zweite dentale Mehrkomponentensystem zu einem Produkt härtbar ist, wobei der durch Härtung (Polymerisationshärtung) maximal erreichbare Druckmodul im Bereich von 100 MPa bis 420 MPa liegt,
   - optional ein drittes dentales Gemisch oder ein drittes dentales Mehrkomponentensystem wie in einem der Ansprüche 1 oder 2 definiert, wobei das dritte dentale Gemisch bzw. das dritte dentale Mehrkomponentensystem zu einem Produkt härtbar ist, wobei der durch Härtung (Polymerisationshärtung) maximal erreichbare Druckmodul kleiner ist als 25 MPa,
   - optional ein weiteres dentales Gemisch oder ein weiteres dentales Mehrkomponentensystem, wobei das weitere dentale Gemisch bzw. das weitere dentale Mehrkomponentensystem zu einem Produkt härtbar ist, wobei der durch Härtung (Polymerisationshärtung) maximal erreichbare Druckmodul größer ist als 420 MPa.

11. Verfahren zur Herstellung einer provisorischen Suprakonstruktion für ein dentales Implantat oder eines Kerns für eine provisorische Suprakonstruktion für ein dentales Implantat nach einem der Ansprüche 3 bis 6 oder eines provisorischen Zahnersatzes nach einem der Ansprüche 7 oder 8, mit folgenden Schritten:

- Bereitstellen oder Herstellen eines dentalen Gemisches oder eines dentalen Mehrkomponentensystems nach einem der Ansprüche 1 oder 2,
- Härten dentalen Gemisches oder des dentalen Mehrkomponentensystems, vorzugsweise in einer Form,
- optional weitere Schritte,

oder

- Bereitstellen eines Kits nach Anspruch 9 umfassend Rohlinge,
- Anpassen der Form eines der Rohlinge,

so dass die provisorische Suprakonstruktion, der Kern oder der provisorische Zahnersatz resultiert.

## Claims

1. Dental mixture or dental multicomponent system for producing the material of a provisional suprastructure for a dental implant or a core for a provisional suprastructure for a dental implant by polymerisation curing, consisting of

    (a) a total of 20.0 to 99.9 per cent by weight of one, two or more polymerisable (meth)acrylates,
    (b) a total of 0.1 to 10.0 per cent by weight of one or more catalysts and/or initiators for the polymerisation,
    (c) a total of 0.0 to 60.0 per cent by weight of one or more inorganic and/or organic fillers,
    (d) a total of 0.0 to 5.0 per cent by weight of one or more stabilisers for which: the stabiliser or stabilisers are not polymerisable (meth)acrylates, and
    (e) a total of 0.0 to 50.0 per cent by weight of one or more further additives,

    wherein the dental mixture or the multicomponent system can be cured to give a product,
    for use in a therapeutic treatment to accelerate the osseointegration of dental implants by the method of progressive bone loading, wherein

    - a provisional suprastructure for a dental implant or a core for a provisional suprastructure for a dental implant is produced from the dental mixture or the multicomponent system by polymerisation curing

    or

    - in the context of the treatment several provisional suprastructures consisting of or as core material comprising materials of different compressive modulus are produced and are joined to the dental implant consecutively in the sequence of increasing compressive moduli.

2. Dental mixture or dental multicomponent system for use in a therapeutic treatment to accelerate the osseointegration of dental implants by the method of progressive bone loading according to claim 1, comprising in or as component (c) one or more particulate inorganic and/or organic fillers, preferably selected from the group consisting of silicon dioxide, metal oxides and poly(meth)acrylates.

3. Provisional suprastructure for a dental implant or core for a provisional suprastructure for a dental implant, producible by polymerisation curing of a dental mixture or a multicomponent system as defined in one of claims 1 or 2.

4. Provisional suprastructure or core for a provisional suprastructure according to claim 3, wherein the material of the suprastructure or the material of the core has a compressive modulus of less than 420 MPa.

5. Provisional suprastructure according to one of claims 3 or 4, consisting of a core and a cap for the core, wherein the outer surface of the core is partially exposed, so that the core is deformable under a compressive load at least in sections independently of the cap, wherein the material of the cap preferably has a higher compressive modulus than the material of the core.

6. Provisional suprastructure according to one of claims 3 to 5, producible by an individual polymerisation curing of an individual dental mixture as defined in one of claims 1 or 2.

7. Provisional dental prosthesis comprising

- a provisional suprastructure according to one of claims 3 to 6,
- a dental implant

and

- a preferably metallic abutment which is arranged between the provisional suprastructure and dental implant and ensures joining thereof

and optionally

- an adhesion promoter which is arranged between the provisional suprastructure and the abutment.

8. Provisional dental prosthesis according to claim 7, wherein the provisional suprastructure and the abutment are joined without positive locking in the force direction along the longitudinal axis of the dental implant away from the jaw bone into which the implant is inserted or to be inserted.

9. Kit for producing a plurality of provisional suprastructures for a dental implant or a plurality of cores for provisional suprastructures for a dental implant, comprising

- a corresponding number of dental mixtures or dental multicomponent systems as defined in one of claims 1 or 2, wherein each of the dental mixtures or multicomponent systems can be cured to give a product, wherein the compressive modulus of the products under the same curing conditions differs

or

- a corresponding number of blanks from a cured or producible by curing from a dental mixture as defined in one of claims 1 or 2, wherein the compressive modulus of the material of each of the blanks differs and is in each case 420 MPa or less,

and

- optionally one or more further constituents selected from the group consisting of adhesion promoters, abutments, implants, moulding tools (moulds), impression materials.

10. Kit according to claim 9, comprising

- a first dental mixture or a first dental multicomponent system as defined in one of claims 1 or 2, wherein the first dental mixture or the first dental multicomponent system can be cured to give a product, wherein the maximum compressive modulus which can be achieved by curing (polymerisation curing) is in the range of from 25 MPa to less than 100 MPa,
- a second dental mixture or a second dental multicomponent system as defined in one of claims 1 or 2, wherein the second dental mixture or the second dental multicomponent system can be cured to give a product, wherein the maximum compressive modulus which can be achieved by curing (polymerisation curing) is in the range of from 100 MPa to 420 MPa,
- optionally a third dental mixture or a third dental multicomponent system as defined in one of claims 1 or 2, wherein the third dental mixture or the third dental multicomponent system can be cured to give a product, wherein the maximum compressive modulus which can be achieved by curing (polymerisation curing) is less than 25 MPa,
- optionally a further dental mixture or a further dental multicomponent system, wherein the further dental mixture or the further dental multicomponent system can be cured to give a product, wherein the maximum compressive modulus which can be achieved by curing (polymerisation curing) is greater than 420 MPa.

11. Method for producing a provisional suprastructure for a dental implant or a core for a provisional suprastructure for a dental implant according to one of claims 3 to 6 or a provisional dental prosthesis according to one of claims 7 or 8, having the following steps:

- providing or producing a dental mixture or a dental multicomponent system according to one of claims 1 or 2,
- curing the dental mixture or the dental multicomponent system, preferably in a mould,

- optionally further steps,

or

- providing a kit according to claim 9 comprising blanks
- adapting the shape of one of the blanks,

so that the provisional suprastructure, the core or the provisional dental prosthesis results.

**Revendications**

1. Mélange dentaire ou système à plusieurs composants dentaire servant à fabriquer le matériau d'une superstructure provisoire pour un implant dentaire ou d'un noyau pour une superstructure provisoire pour un implant dentaire par durcissement par polymérisation, constitué

   (a) au total de 20,0 à 99,9 pour cent en poids d'un, de deux (méth)acrylates polymérisables ou plus,
   (b) au total de 0,1 à 10,0 pour cent en poids d'un ou de plusieurs catalyseurs et/ou initiateurs pour la polymérisation,
   (c) au total de 0,0 à 60,0 pour cent en poids d'une ou de plusieurs charges inorganiques et/ou organiques,
   (d) au total de 0,0 à 5,0 pour cent en poids d'un ou de plusieurs stabilisateurs, pour lesquels s'applique ce qui suit : le ou les stabilisateurs ne sont pas des (méth)acrylates polymérisables, ainsi
   (e) au total de 0,0 à 50,0 pour cent en poids d'un ou de plusieurs additifs,

   dans lequel le mélange dentaire ou le système à plusieurs composants peut être durci en un produit,
   à utiliser lors d'un traitement thérapeutique servant à accélérer l'ostéo-intégration d'implants dentaires d'après la technique de l'allongement osseux progressif (progressive bone loading), dans lequel

   - une superstructure provisoire pour un implant dentaire ou un noyau pour une superstructure provisoire pour un implant dentaire est fabriqué(e) par durcissement par polymérisation à partir du mélange dentaire ou du système à plusieurs composants

   ou

   - dans le cadre du traitement, plusieurs superstructures provisoires constituées de matériaux ou comprenant, en tant que matériau de noyau, des matériaux sont fabriquées avec différents modules de pression et sont reliées les unes après les autres à l'implant dentaire dans l'ordre de modules de pression croissante.

2. Mélange dentaire ou système à plusieurs composants dentaire destiné à être utilisé dans un traitement thérapeutique servant à accélérer l'ostéo-intégration d'implants dentaires selon la technique de l'allongement osseux progressif selon la revendication 1, comprenant, dans ou en tant que composant (c), une ou plusieurs charges particulaires inorganiques et/ou organiques, de préférence choisies parmi le groupe constitué de dioxyde de silicium, d'oxydes métalliques et de poly(méth)acrylates.

3. Superstructure provisoire pour un implant ou un noyau dentaire pour une superstructure provisoire pour un implant dentaire, pouvant être fabriquée par durcissement par polymérisation d'un mélange dentaire ou d'un système à plusieurs composants tels que définis dans l'une quelconque des revendications 1 ou 2.

4. Superstructure provisoire ou noyau pour une superstructure provisoire selon la revendication 3, dans laquelle le matériau de la superstructure ou le matériau du noyau possède un module de pression inférieure à 420 MPa.

5. Superstructure provisoire selon l'une quelconque des revendications 3 ou 4, constituée d'un noyau et d'une coiffe pour le noyau, dans laquelle la surface extérieure du noyau est en partie dégagée de sorte que le noyau puisse être déformé au moins par endroits indépendamment de la coiffe en cas d'application d'une pression, dans laquelle le matériau de la coiffe présente de préférence un module de pression plus élevée que le matériau du noyau.

6. Superstructure provisoire selon l'une quelconque des revendications 3 à 5, pouvant être fabriquée par un unique durcissement par polymérisation d'un unique mélange dentaire tel que défini dans l'une quelconque des revendications 1 ou 2.

**7.** Prothèse dentaire provisoire comprenant

- une superstructure provisoire selon l'une quelconque des revendications 3 à 6,
- un implant dentaire

et

- un pilier, de préférence métallique, qui est disposé entre la superstructure provisoire et l'implant dentaire et qui garantit leur liaison,

ainsi qu'en option

- un promoteur d'adhérence, qui est disposé entre la superstructure provisoire et le pilier.

**8.** Prothèse dentaire provisoire selon la revendication 7, dans laquelle la superstructure provisoire et le pilier sont reliés sans complémentarité de forme, dans la direction de force le long de l'axe longitudinal de l'implant dentaire, de manière éloignée de l'os de la mâchoire, dans lequel l'implant est ou doit être inséré.

**9.** Ensemble servant à fabriquer une multitude de superstructures provisoires pour un implant dentaire ou une multitude de noyaux pour des superstructures provisoires pour un implant dentaire, comprenant

- un nombre correspondant de mélanges dentaires ou de systèmes à plusieurs composants dentaires tels que définis dans l'une quelconque des revendications 1 ou 2, dans lequel chacun des mélanges ou systèmes à plusieurs composants dentaires peut être durci en un produit, dans lequel le module de pression des produits est différent en présence de conditions de durcissements identiques

ou

- un nombre correspondant d'ébauches composées d'un mélange dentaire durci ou pouvant être fabriquées par durcissement à partir d'un mélange dentaire tel que défini dans l'une quelconque des revendications 1 ou 2, dans lequel le module de pression du matériau de chacune des ébauches est différent et présente une pression respectivement égale à 420 MPa ou inférieure,

ainsi

- qu'en option un ou plusieurs autres composants choisis parmi le groupe constitué des promoteurs d'adhérence, de piliers, d'implants, d'outils de moulage (moules), de matériaux à empreinte.

**10.** Ensemble selon la revendication 9, comprenant

- un premier mélange dentaire ou un premier système à plusieurs composants dentaire tels que définis dans l'une quelconque des revendications 1 ou 2, dans lequel le premier mélange dentaire ou le système à plusieurs composants dentaire peut être durci en un produit, dans lequel le module de pression pouvant être atteint au maximum par durcissement (durcissement par polymérisation) présente une pression se situant dans la plage allant de 25 MPa à une pression inférieure à 100 MPa,
- un deuxième mélange dentaire ou un deuxième système à plusieurs composants dentaire tels que définis dans l'une quelconque des revendications 1 ou 2, dans lequel le deuxième mélange dentaire ou le deuxième système à plusieurs composants dentaire peut être durci en un produit, dans lequel le module de pression pouvant être atteint au maximum par durcissement (durcissement par polymérisation) présente une pression se situant dans la plage allant de 100 MPa jusqu'à 420 MPa,
- en option un troisième mélange dentaire ou un troisième système à plusieurs composants dentaire tels que définis dans l'une quelconque des revendications 1 ou 2, dans lequel le troisième mélange dentaire ou le troisième système à plusieurs composants dentaire peut être durci en un produit, dans lequel le module de pression pouvant être atteint au maximum par durcissement (durcissement par polymérisation) présente une pression inférieure à 25 MPa,
- en option un autre mélange dentaire ou un autre système à plusieurs composants dentaire, dans lequel l'autre mélange dentaire ou l'autre système à plusieurs composants dentaire peut être durci en un produit, dans lequel le module de pression pouvant être atteint au maximum par durcissement (durcissement par polymérisation) présente une pression supérieure à 420 MPa.

11. Procédé servant à fabriquer une superstructure provisoire pour un implant dentaire ou un noyau pour une superstructure dentaire pour un implant dentaire selon l'une quelconque des revendications 3 à 6 ou une prothèse dentaire provisoire selon l'une quelconque des revendications 7 ou 8, comprenant des étapes qui suivent consistant à :

- fournir ou fabriquer un mélange dentaire ou un système à plusieurs composants dentaire selon l'une quelconque des revendications 1 ou 2,
- faire durcir le mélange dentaire ou le système à plusieurs composants dentaire de préférence dans un moule,
- en option, mettre en oeuvre d'autres options,

ou

- fournir un ensemble selon la revendication 9 comprenant des ébauches,
- adapter la forme d'une des ébauches,

de sorte que la superstructure provisoire, le noyau ou la prothèse dentaire provisoire en résultent.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 270915 A
- EP 630640 A
- US 6063830 A
- US 5548001 A
- US 4617327 A
- EP 0677286 A
- US 4387215 A
- DE 19506222
- DE 4439485
- US 5665839 A
- DE 19612004
- DE 102004002178
- WO 2008037753 A2
- US 7798812 A1
- DE 102006019092 A1
- DE 3941629 C2
- DE 102006019092
- DE 60116142
- DE 3801511 C2
- DE 102006050153 A1
- EP 0184095 B1
- DE 4231579 C2
- EP 0366977 B1
- US 7081485 B2
- DE 3236026 A1
- US 20070027229 A1
- EP 0262629 B1
- EP 0073413 A
- US 7148382 B2
- US 5761169 A
- DE 19708294 A1
- EP 0057474 A
- EP 0047902 A
- EP 0007508 A
- DE 60029481 T2
- EP 0980682 B1
- EP 0948955 B1
- EP 1236459 B1
- EP 0173567 A2
- US 4772530 A
- US 4954414 A
- US 4874450 A
- US 5055372 A
- US 5057393 A
- EP 1720506 A
- EP 1839640 A
- DE 1495520
- WO 02092021 A
- WO 02092023 A
- EP 0059451 A
- EP 1872767 A
- EP 2070506 A
- DE 112006001049
- US 7214726 B
- EP 2070935 A
- WO 2007131725 A
- EP 0783880 B1
- DE 10119831 A1
- EP 1563821 A1

**In der Beschreibung aufgeführte Nicht-Patentliteratur**

- **M. LORENZONI ; C. PERTL ; K. ZHANG ; W. WEG-SCHNEIDER.** *Clin. Oral Implants Res.,* 2003, vol. 14 (3), 273-279
- **P. QUINLAN et al.** *Int. J. Oral Maxillofac Implants,* 2005, vol. 20 (3), 360-370
- **G. ROMANOS.** *J. Oral Implantol.,* 2004, vol. 30 (3), 189-197
- **R. APPLETON et al.** *Clin. Oral Implants Res.,* 2005, vol. 16 (2), 161-167
- **ERICH KARSTEN.** Lackrohstoff-Tabellen. Vincentz Verlag Hannover, 2000, vol. II
- Polymer Handbook. Wiley Verlag, 1999, vol. III
- Chemistry & Technology of UV&EB Formulation for Coatings, Inks & Paints. Prepolymers & Reactive Dilutents. SITA Technology Ltd, 1997, vol. III
- **J.-P. FOUASSIER.** Photoinitiation, Photopolymerization and Photocuring. Hanser Publishers, 1995
- Radiation Curing in Polymer Science and Technology. Elsevier Applied Science, 1993, vol. II